(19) Europäisches Patentamt
European Patent Office
Office européen des brevets

(11) EP 0 756 894 B1

(12) # EUROPÄISCHE PATENTSCHRIFT

(45) Veröffentlichungstag und Bekanntmachung des Hinweises auf die Patenterteilung:
**26.01.2000 Patentblatt 2000/04**

(51) Int Cl.⁷: **B01J 23/88**

(21) Anmeldenummer: **96112063.1**

(22) Anmeldetag: **26.07.1996**

(54) **Multimetalloxidmassen**

Multimetal oxide masses

Masses d'oxydes multimétalliques

(84) Benannte Vertragsstaaten:
**BE DE DK ES FR GB IT NL**

(30) Priorität: **04.08.1995 DE 19528646**

(43) Veröffentlichungstag der Anmeldung:
**05.02.1997 Patentblatt 1997/06**

(73) Patentinhaber: **BASF Aktiengesellschaft**
**67063 Ludwigshafen (DE)**

(72) Erfinder:
- **Hibst, Hartmut, Prof. Dr.**
  **69198 Schriesheim (DE)**
- **Tenten, Andreas, Dr.**
  **67487 Maikammer (DE)**
- **Marosi, Laszlo, Dr.**
  **67063 Ludwigshafen (DE)**

(56) Entgegenhaltungen:
**EP-A- 0 575 897**
**EP-A- 0 668 102**
**EP-A- 0 668 103**
**DE-A- 4 405 059**
**DE-A- 4 407 020**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 756 894 B1

## Beschreibung

Die Erfindung betrifft Multimetalloxidmassen der allgemeinen Formel I

$$[A]_p\ [B]_q \qquad (I),$$

in der die Variablen folgende Bedeutung haben:

| | |
|---|---|
| A | $Mo_{12}\ V_a\ X^1_b\ X^2_c\ X^3_d\ X^4_e\ X^5_f\ X^6_g\ O_x$ (Aktivphase), |
| B | $X^7_{12}Cu_h\ H_i\ O_y$ (Promotorphase), |
| $X^1$ | W, Nb, Ta, Cr und/oder Ce, vorzugsweise W, Nb und/oder Cr, |
| $X^2$ | Cu, Ni, Co, Fe, Mn und/oder Zn, vorzugsweise Cu, Ni, Co und/oder Fe, |
| $X^3$ | Sb und/oder Bi, vorzugsweise Sb, |
| $X^4$ | Li, Na, K, Rb, Cs und/oder H, vorzugsweise Na und/oder K, |
| $X^5$ | Mg, Ca, Sr und/oder Ba, vorzugsweise Ca, Sr und/oder Ba, |
| $X^6$ | Si, Al, Ti und/oder Zr, vorzugsweise Si, Al und/oder Ti, |
| $X^7$ | Mo, W, V, Nb und/oder Ta, vorzugsweise Mo und/oder W, |
| a | 1 bis 8, vorzugsweise 2 bis 6, |
| b | 0,2 bis 5, vorzugsweise 0,5 bis 2,5, |
| c | 0 bis 23, vorzugsweise 0 bis 4, |
| d | 0 bis 50, vorzugsweise 0 bis 3, |
| e | 0 bis 2, vorzugsweise 0 bis 0,3, |
| f | 0 bis 5, vorzugsweise 0 bis 2, |
| g | 0 bis 50, vorzugsweise 0 bis 20, |
| h | 4 bis 30, vorzugsweise 6 bis 24, besonders bevorzugt 9 bis 17, |
| i | 0 bis 20, vorzugsweise 0 bis 10, |
| x,y | Zahlen, die durch die Wertigkeit und Häufigkeit der von Sauerstoff verschiedenen Elemente in I bestimmt werden und |
| p,q | von Null verschiedene Zahlen, deren Verhältnis p/q 160:1 bis 1:1, vorzugsweise 20:1 bis 1:1 und besonders bevorzugt 15:1 bis 4:1 beträgt, |

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A $Mo_{12}\ V_a\ X^1_b\ X^2_c\ X^3_d\ X^4_e\ X^5_f\ X^6_g\ O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B $X^7_{12} Cu_h H_i O_y$

enthalten, wobei die Bereiche A, B relativ zueinander wie in einem Gemisch aus feinteiligem A und feinteiligem B verteilt sind, mit der Maßgabe, daß die Bereiche B Kristallite von Oxometallaten der allgemeinen Formel II

$$Cu\, Mo_A\, W_B\, V_C\, Nb_D\, Ta_E\, O_Y \qquad (II),$$

mit

1/(A+B+C+D+E) 0,7 bis 1,3,vorzugsweise 0,85 bis 1,15, besonders bevorzugt 0,95 bis 1,05 und ganz besonders bevorzugt 1,

(B+C+D+E)/A 0,01 bis 1, vorzugsweise 0,05 bis 0,3, besonders bevorzugt 0,075 bis 0,15 und ganz besonders bevorzugt 0,11 und

Y eine Zahl, die durch die Wertigkeit und Häufigkeit der von Sauerstoff verschiedenen Elemente zu II bestimmt wird,

eines neuen Strukturtyps enthalten, der nachfolgend durch sein Röntgenbeugungsmuster (Fingerabdruck) definiert wird. Wiedergegeben in Gestalt von von der Wellenlänge der verwendeten Röntgenstrahlung unabängigen Netzebenenabständen d[Å] sind die charakteristischsten und intensivsten Beugungslinien des neuen Strukturtyps:

| d [Å] | Intensität [%] |
|---|---|
| 3,40 ± 0,3 | 100 |
| 3,54 ± 0,3 | 72 |
| 2,27 ± 0,2 | 39 |
| 6,79 ± 0,3 | 32 |
| 2,56 ± 0,2 | 25 |
| 1,57 ± 0,2 | 22 |
| 1,87 ± 0,2 | 19 |
| 2,96 ± 0,3 | 18 |
| 3,56 ± 0,3 | 18 |
| 1,64 ± 0,2 | 17 |
| 2,66 ± 0,2 | 16 |
| 1,59 ± 0,2 | 16 |
| 1,55 ± 0,2 | 16 |
| 2,67 ± 0,2 | 14 |
| 2,00 ± 0,2 | 14 |
| 3,04 ± 0,3 | 13 |
| 1,57 ± 0,2 | 11 |
| 2,36 ± 0,2 | 11 |
| 1,44 ± 0,2 | 11 |
| 1,70 ± 0,2 | 10 |

(fortgesetzt)

| d [Å] | Intensität [%] |
|---|---|
| 1,51 ± 0,2 | 10 |
| 2,35 ± 0,2 | 10 |

Die Intensitätsangaben sind relative Bezugswerte und auf die intensitätsstärkste Beugungslinie bezogen. Unter Intensität wird hier die maximale Amplitude der Röntgenbeugungsbande verstanden. Die zugehörigen Beugungswinkel $\Theta$ ergeben sich aus der Bragg'schen Beziehung:

$$\sin\Theta = \lambda/2d,$$

wobei $\lambda$ die Wellenlänge der zur Röntgenbeugung verwendeten Röntgenstrahlung ist. Vorstehende Angaben gehen zurück auf eine Pulveraufnahme an einem Oxometallat $Cu_1\ Mo_{0,9}\ W_{0,1}\ O_4$. Die entsprechende Röntgenaufnahme wurde mit einem Siemens Diffraktometer D-5000 unter Anwendung von Cu-K$\alpha$-Strahlung (40 kV, 30 mA, $\lambda$ = 1,5406 Å) erzeugt. Das Diffraktometer war mit automatischer Divergenz-, Streustrahl- und Zählrohrblende sowie einem Peltier-Detektor ausgerüstet. Bezüglich der Angabe der Linienintensitäten sei vermerkt, daß die relativen Linienintensitäten, im Unterschied zur Lage der Linien, durch die sich bei verschiedenen Pulveraufnahmepräparationen, fußend auf der Anisotropie der Kristallform, einstellenden individuellen Kristallitausrichtungen in dem Fachmann an sich bekannter Weise merklich beeinflußt werden und daher zu Identifikation des neuen Strukturtyps eine geringere Signifikanz aufweisen. Vorstehende quantitative Intensitätsangaben sind daher als typische Werte zu verstehen, die bei den intensitätsstärksten Linien (30-100 %, bezogen auf die intensitätsstärkste Linie) um bis zu ± 50 % (bezogen auf die angegebenen Werte) variieren können.

Nachfolgend soll der neue Strukturtyp als HT (High Temperature)-Cu-Molybdat-Typ bezeichnet und Kristallite aus Oxometallaten II des vorstehend definierten neuen Strukturtyps als Kristallite B* gekennzeichnet werden.

Außerdem betrifft vorliegende Erfindung die Oxometallate II selbst und Verfahren zur Herstellung von Oxometallaten II sowie von Multimetalloxidmassen I.

Desweiteren betrifft vorliegende Erfindung die Verwendung von Oxometallaten II bzw. Multimetalloxidmassen I für katalytische Gasphasenoxidationen niedermolekularer organischer Verbindungen.

Die DE-A 4 335 973 und die US-A 4 035 262 betreffen Multimetalloxidmassen, deren Element-Bruttozusammensetzung derjenigen der erfindungsgemäßen Multimetalloxidmassen entspricht. Die Herstellung dieser Multimetalloxidmassen erfolgt dadurch, daß man geeignete Quellen der Bestandteile der gewünschten Multimetalloxidmassen in den erforderlichen Mengen zu einem innigen Trockengemisch verarbeitet und dieses anschließend bei erhöhter Temperatur mehrere Stunden calciniert. Die resultierenden Multimetalloxidmassen werden als Katalysatoren zur gasphasenkatalytisch oxidativen Herstellung von Acrylsäure aus Acrolein empfohlen. Nachteilig an den Multimetalloxidmassen dieses Standes der Technik ist jedoch, daß bei ihrer Verwendung die Selektivität der Acrylsäurebildung bei vorgegebenem Acroleinumsatz nicht voll zu befriedigen vermag. Ferner weisen diese Multimetalloxidmassen ein ausgeprägtes Formierungsverhalten auf. D.h. bei Verwendung von frisch hergestellten Multimetalloxidmassen erreicht die Selektivität (bei vorgegebenem Acroleinumsatz) der Acrylsäurebildung erst nach längerer Betriebsdauer ihren dann im wesentlichen stationären Endwert. Auch vermag die Reproduzierbarkeit ihrer Herstellung bezüglich des stationären Endwertes der Selektivität der Acrylsäurebildung nicht zu befriedigen.

Die EP-A 835, die DE-C 3 338 380, die DE-A 4 220 859 und die DE-A 4 307 381 betreffen ebenfalls als Katalysatoren für die gasphasenkatalytisch oxidative Herstellung $\alpha,\beta$-monoethylenisch ungesättigter Carbonsäuren geeignete Multimetalloxidmassen, die in vorteilhafter Weise einen Co-Phase/Schlüsselphase-Aufbau aufweisen. Zwar umfassen die allgemeinen Formeln dieses Standes der Technik innerhalb einer breiten Mannigfaltikeit möglicher Multimetalloxidmassen formal auch solche, deren Schlüsselphase neben Elementen wie Molybdän oder Wolfram gleichzeitig das Element Kupfer enthalten können, die Gesamtheit aller Ausführungsbeispiele umfaßt jedoch kein einziges solches Ausführungsbeispiel, vielmehr sind selbige auf solche beschränkt, deren Schlüsselphase anstelle des Elements Kupfer das Element Wismut enthalten. Diese Ausführungsform empfiehlt der Stand der Technik nachdrücklich als die besonders bevorzugte. Nachteilig an dieser bevorzugten Ausführungsform des Standes der Technik ist jedoch, daß auch sie als Katalysator für die katalytische Gasphasenoxidation von Acrolein zu Acrylsäure hinsichtlich der Selektivität der Acrylsäurebildung bei vorgegebenem Acroleinumsatz nicht voll zu befriedigen vermag.

Die älteren Anmeldungen DE-A 44 05 058, DE-A 44 05 059 und DE-A 44 05 060 betreffen Multimetalloxidmassen, die ebenfalls einen Co-Phase/Schlüsselphase-Aufbau aufweisen. Sie eignen sich insbesondere zur gasphasenkatalytisch oxidativen Herstellung von Methacrylsäure, vermögen jedoch für die katalytische Gasphasenoxidation

von Acrolein zu Acrylsäure hinsichtlich der Selektivität der Acrylsäurebildung bei vorgegebenem Acroleinumsatz nicht zu befriedigen.

Die ältere Anmeldung DE-A 44 05 514 und die ältere Anmeldung DE-A 44 40 891 betreffen Multimetalloxidmassen, die ebenfalls einen Co-Phase/Schlüsselphase-Aufbau $[A']_p$, $[B']_q$, aufweisen, wobei die dortigen Bruttoelementzusammensetzungen der Co-Phase und der Schlüsselphase mit den Bruttoelementzusammensetzungen der Aktivphase und der Promotorphase der Multimetalloxidmassen I der vorliegenden Erfindung jeweils übereinstimmen. Die beiden vorgenannten älteren Anmeldungen führen ferner aus, daß die dortigen Schlüsselphasen B' Kristallite enthalten können, die den Strukturtyp (das Röntgenbeugungsmuster) wenigstens eines der in der nachfolgenden Tabelle 1 aufgelisteten Kupfermolybdate aufweisen (der Ausdruck in Klammern gibt die Quelle für den zugehörigen Röntgenbeugungsfingerabdruck wieder):

Tabelle 1

| | |
|---|---|
| $Cu_3 (MoO_4)_2 (OH)_2$ | (Lindgrenit, Karteikarte 36-405 der JCPDS-ICDD Kartei (1991)), |
| $Cu_4 MoO_6 O_{20}$ | (A. Moini et al., Inorg. Chem. 25 (21) (1986) S. 3782 bis 3785), |
| $Cu_4 Mo_5 O_{17}$ | (Karteikarte 39-181 der JCPDS-ICDD Kartei (1991)), |
| $Cu_6 Mo_5 O_{18}$ | (Karteikarte 40-865 der JCPDS-ICDD Kartei (1991)), |
| $Cu_6 Mo_4 O_{15}$ | (Karteikarte 35-17 der JCPDS-ICDD Kartei (1991)), |
| $Cu Mo O_4$ | (Karteikarte 22-242 der JCPDS-ICDD Kartei (1991)), |
| $Cu Mo O_4$ | (Russian Journal of Inorganic Chemistry 36 (7), 1991, S. 927-928, Table 1, Cu Mo $O_4$-III mit verzerrter Wolframit-Struktur ($CuWO_4$, Karteikarte 21-307 JCPDS-ICDD Kartei (1994)), |
| $Cu_{4-x} Mo_3 O_{12}$ | mit x = O bis 0,25 (Karteikarte 24-56 und 26-547 der JCPDS-ICDD Kartei (1991)), |
| $Cu_3 Mo_2 O_9$ | (Karteikarte 24-55 und 34-637 der JCPDS-ICDD Kartei (1991)), |
| $Cu_2 Mo O_5$ | (Karteikarte 22-607 der JCPDS-ICDD Kartei (1991)). |

Als für die katalytische Gasphasenoxidation von Acrolein zu Acrylsäure besonders günstige Multimetalloxid-Katalysatoren $[A']_p$, $[B']_q$, empfehlen die DE-A 44 05 514 und die DE-A 44 40 891 solche Multimetalloxidmassen, deren Schlüsselphase Kristallite von Oxometallaten der allgemeinen Formel III

$$CuMo_{A'} W_{B'} V_{C'} Nb_{D'} Ta_{E'} O_{Y'} \cdot (H_2O)_{F'} \qquad \text{(III)},$$

mit

| | |
|---|---|
| 1/(A'+B'+C'+D'+E') | 0,7 bis 1,3, |
| F' | 0 bis 1, |
| B'+C'+D'+E' | 0 bis 1 und |
| Y' | eine Zahl, die durch die Wertigkeit und Häufigkeit der von Sauerstoff verschiedenen Elemente in III bestimmt wird, |

des Strukturtyps enthalten, der durch die Verbindung $CuMoO_4$-III in Russian Journal of Inorganic Chemistry 36(7), 1991 auf S. 921 in Table 1 definiert wird und in den DE-As als Wolframit bezeichnet wurde.

Zum Zeitpunkt der Anmeldung der DE-A 44 05 514 sowie der DE-A 44 40 891 nicht bekannt war die Tatsache, daß es den HT-Cu-Molybdat-Strukturtyp überhaupt gibt, geschweige denn, daß spezielle Oxometallate III, nämlich die

Oxometallate II, in diesem Strukturtyp auftreten. Ebenso unbekannt war die Tatsache, daß die erfindungsgemäßen Multimetalloxidmassen I, deren Promotorphase B Kristallite von Oxometallaten II im HT-Cu-Molybdat-Strukturtyp aufweist, sich in vorteilhafter Weise insbesondere als Katalysatoren zur gasphasenkatalytischen Oxidation von Acrolein zu Acrylsäure eignen.

Demgemäß bildet das Vorgenannte den Gegenstand der vorliegenden Erfindung.

Ganz besonders bevorzugte Massen I sind solche, deren Bereiche A eine Zusammensetzung der nachfolgenden allgemeinen Formel IV aufweisen

$$Mo_{12}\,V_{a'}X^1_{b'}X^2_{c'}X^5_{f'}X^6_{g'}O_{x'} \qquad (IV),$$

mit

- $X^1$ W und/oder Nb,
- $X^2$ Cu und/oder Ni,
- $X^5$ Ca und/oder Sr,
- $X^6$ Si und/oder Al,
- a' 2 bis 6,
- b' 1 bis 2,
- c' 1 bis 3,
- f' 0 bis 0,75,
- g' 0 bis 10 und
- x' eine Zahl, die durch die Wertigkeit und Häufigkeit der von Sauerstoff verschiedenen Elemente in IV bestimmt wird.

Ferner ist es von Vorteil, wenn der Anteil $[B]_q$ der erfindungsgemäßen Multimetalloxidmassen I in den letzteren in Form dreidimensional ausgedehnter Bereiche der chemischen Zusammensetzung B enthalten ist, deren Größtdurchmesser $d_B$ (längste durch den Schwerpunkt des Bereichs gehende Verbindungsstrecke zweier auf der Oberfläche (Grenzfläche) des Bereichs befindlicher Punkte) > 0 bis 300 μm, vorzugsweise 0,1 bis 200 μm, besonders bevorzugt 0,5 bis 50 μm und ganz besonders bevorzugt 1 bis 30 μm betragen. Selbstverständlich können die Größtdurchmesser aber auch 50 bis 150 μm oder 75 bis 125 μm betragen (die experimentelle Ermittlung der Größtdurchmesser gestattet z.B. die Methode der lateral aufgelösten energiedispersiven Röntgenanalyse (EDXS) z.B. mittels einer Elektronenstrahl-Mikrosonde JEOL JCXA/733).

Der Anteil $[A]_p$ kann in den erfindungsgemäßen Multimetalloxidmassen I amorph und/oder kristallin vorliegen. Der Anteil $[B]_q$ kann ausschließlich aus Oxometallat-II-Kristalliten des neuen Strukturtyps bestehen, als auch Kristallite einer Zusammensetzung

$$X^7_{12}Cu_h\,H_i\,O_y$$

eines (oder mehrerer) anderen Strukturtyps, insbesondere der in Tabelle 1 aufgeführten, umfassen. Selbstverständlich kann der Anteil $[B]_q$ auch amorphe Zusammensetzungen

$$X^7_{12}Cu_h\,H_i\,O_y$$

enthalten.

Bezogen auf die Gesamtmasse des Anteils $[B]_q$, kann sich der Anteil der Kristallite B* daher auf > 0 bis 100, oder 1 bis 95, oder 5 bis 90, oder 10 bis 85, oder 15 bis 75, oder 25 bis 65, oder 35 bis 55, oder 40 bis 50 Gew.-% belaufen. Geeignet ist selbstverständlich auch ein Gewichtsanteil von 95 bis 100 Gew.-%.

Erfindungsgemäß besonders günstige Kristallite B* sind solche der Stöchiometrie II mit C+D+E=O. D.h., erfindungsgemäß günstige Oxometallate II des neuen Strukturtyps sind solche der allgemeinen Formel V

$$Cu\,Mo_A\,W_B\,O_Y \qquad (V),$$

mit

1/(A+B) 0,7 bis 1,3, vorzugsweise 0,85 bis 1,15, besonders bevorzugt 0,09 bis 1,05 und ganz besonders bevorzugt 1,

B/A 0,01 bis 1,vorzugsweise 0,05 bis 0,2, besonders bevorzugt 0,075 bis 0,15 und ganz besonders bevorzugt 0,11,

Y eine Zahl, die durch die Wertigkeit und Häufigkeit der von Sauerstoff verschiedenen Elementen in V bestimmt wird, häufig 3,5 bis 4.

D.h. ein ganz besonders günstiges erfindungsgemäßes Oxometallat II des neuen Strukturtyps weist die Zusammensetzung

$$Cu_1\ Mo_{0,9}\ W_{0,1}O_{3,5\text{-}4}$$

auf.

Die erfindungsgemäßen Massen I sind in einfacher Weise z.B. dadurch erhältlich, daß man eine Multimetalloxidmasse

$$X^7_{12}Cu_h\ H_i\ O_y \qquad \text{(B)},$$

die Kristallite von Oxometallaten II des neuen Strukturtyps enthält oder ausschließlich aus solchen besteht, in feinteiliger Form getrennt vorbildet (Ausgangsmasse 1) und anschließend die Ausgangsmasse 1 mit geeigneten Quellen der elementaren Konstituenten der Multimetalloxidmasse A

$$Mo_{12}\ V_a\ X^1_b\ X^2_c\ X^3_d\ X^4_e\ X^5_f\ X^6_g\ O_x \qquad \text{(A)}$$

im gewünschten Mengenverhältnis in innigen Kontakt bringt und ein daraus resultierendes Trockengemisch bei einer Temperatur von 250 bis 500°C calciniert, wobei die Calcination unter Inertgas (z.B. $N_2$), einem Gemisch aus Inertgas und Sauerstoff (z.B. Luft), reduzierend wirkenden Gasen wie Kohlenwasserstoffen (z.B. Methan), Aldehyden (z.B. Acrolein) oder Ammoniak aber auch unter einem Gemisch aus $O_2$ und reduzierend wirkenden Gasen (z.B. allen vorgenannten) erfolgen kann, wie es beispielsweise in der DE-A 4 335 973 beschrieben wird. Bei einer Calcination unter reduzierenden Bedingungen ist zu beachten, daß die metallischen Konstituenten nicht bis zum Element reduziert werden. Die Calcinationsdauer erstreckt sich in der Regel über einige Stunden und nimmt üblicherweise mit zunehmender Calcinierungstemperatur ab. Wesentlich für die Quellen der elementaren Konstituenten der Multimetalloxidmasse A ist dabei, wie allgemein bekannt, nur, daß es sich entweder bereits um Oxide handelt oder um solche Verbindungen, die durch Erhitzen, wenigstens in Anwesenheit von Sauerstoff, in Oxide überführbar sind. Neben den Oxiden kommen als Ausgangsverbindungen vor allem Halogenide, Nitrate, Formiate, Oxalate, Citrate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate) bzw. die von diesen abgeleiteten Säuren.

Kristallite von Oxometallaten II des neuen Strukturtyps können in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 700 bis 900°C, vorzugsweise 700 bis 800°C, mehrere Stunden unter Inertgas oder, bevorzugt, an der Luft, calciniert.

In überraschender Weise entstehen im Rahmen der vorgenannten Hochtemperaturcaldination in der Regel entweder Kristallite von Oxometallaten II des neuen Strukturtyps für sich, oder im Gemisch mit Kristalliten anderer Oxometallat-Strukturtypen.

Das innige Vermischen der Ausgangsverbindungen kann dabei in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach Trocknung calciniert. Vorzugsweise erfolgt der Trocknungsprozeß unmittelbar im Anschluß an die Fertigstellung der wäßrigen Mischung und durch Sprühtrocknung (die Austrittstemperaturen betragen in der Regel 100 bis 150°C), die ein besonders inniges Trockengemisch bedingt.

Besonders innige Trockengemische werden beim beschriebenen Trockenverfahren dann erhalten, wenn ausschließlich von in gelöster Form befindlichen Quellen der elementaren Konstituenten ausgegangen wird. Im Fall des elementaren Konstituenten Kupfer ist es in diesem Zusammenhang besonders vorteilhaft von wäßrigen Lösungen auszugehen, die ihn in Gestalt von Kupfer-Ammoniak-Komplexen (z.B. Tetrammin) gelöst enthalten.

Die wie ebenda beschrieben erhältlichen Kristallite von Oxometallaten II des neuen Strukturtyps oder die sie enthaltenden Kristallitgemische können nun, gegebenenfalls nach Mahlung und/oder Klassierung auf gewünschte Größen, z.B. für sich als Ausgangsmasse 1 eingesetzt werden.

Selbstverständlich können sie aber auch zuvor mit Multimetalloxiden

$$X^7_{12}Cu_h\,H_i\,O_y$$

anderer Strukturtypen, wie sie z.B. in Tabelle 1 aufgelistet und nach den in den DE-A 44 05 514 und DE-A 44 40 891 beschriebenen Verfahren erhältlich sind (Ausgangsmassen 1'), im gewünschten Verhältnis zu einer neuen Ausgangsmasse 1'' abgemischt werden. Letztere kann als solche eingesetzt werden oder bei Bedarf zunächst verpresst, gesintert, dann wieder zerkleinert und danach eingesetzt werden.

Das innige Inkontaktbringen der Ausgangsmasse 1 (bzw. Ausgangsmasse 1'') mit den Quellen der Multimetalloxidmasse A (Ausgangsmasse 2) kann sowohl trocken als auch naß erfolgen. Im letzteren Fall muß lediglich darauf geachtet werden, daß die vorgebildeten Kristallite B* und gegebenenfalls sonstige gewünschte B-Kristallite, nicht in Lösung gehen. In wäßrigem Medium ist letzteres bei pH-Werten, die nicht zu stark von 7 abweichen, üblicherweise gewährleistet. Erfolgt das innige Inkontaktbringen naß, wird anschließend normalerweise zu einer Trockenmasse getrocknet (vorzugsweise durch Sprühtrocknen). Eine solche Trockenmasse fällt im Rahmen eines trockenen Mischens automatisch an. Natürlich kann das innige Inkontaktbringen der "Ausgangsmasse 1''" mit den Quellen der Multimetalloxidmasse A (Ausgangsmasse 2) auch so erfolgen, daß zunächst eine Ausgangsmasse 1 und danach eine Ausgangsmasse 1', oder umgekehrt, in Kontakt gebracht wird.

Als mögliche Mischungsvarianten kommen damit z.B. in Betracht:

a. eine trockene, feinteilige, vorgebildete Ausgangsmasse 1 mit trockenen, feinteiligen Ausgangsverbindungen der elementaren Konstituenten des gewünschten Multimetalloxids A im gewünschten Mengenverhältnis in einem Mischer, Kneter oder in einer Mühle mischen;

b. ein feinteiliges Multimetalloxid A vorbilden durch inniges Mischen geeigneter Ausgangsverbindungen ihrer elementaren Konstituenten (trocken oder naß) und anschließendes Calcinieren der daraus resultierenden innigen Trockenmischung bei Temperaturen von 250 bis 450°C (bezüglich Calcinationsdauer, Calcinationsatmosphäre und Elementquellen gilt das auf Seite 9 gesagte); das vorgebildete Multimetalloxid A feinteilig gestalten und mit der feinteiligen Ausgangsmasse 1 im gewünschten Mengenverhältnis wie in a. mischen; bei dieser Mischungsvariante ist ein abschließendes Calcinieren der resultierenden Mischung nicht essentiell;

c. in eine wäßrige Lösung und/oder Suspension von Ausgangsverbindungen der elementaren Konstituenten des gewünschten Multimetalloxids A die erforderliche Menge vorgebildeter Ausgangsmasse 1 einrühren und anschließend sprühtrocknen; selbstverständlich kann anstelle der Ausgangsverbindungen der elementaren Konstituenten des gewünschten Multimetalloxids A auch ein bereits gemäß b. vorgebildetes Multimetalloxid A selbst eingesetzt werden.

Natürlich können auch alle zwischen a., b. und/oder c. liegenden Mischungsvarianten angewendet werden. Das resultierende innige Trockengemisch kann anschließend wie beschrieben calciniert und danach zur gewünschten Katalysatorgeometrie geformt werden oder umgekehrt. Prinzipiell kann das calcinierte (oder bei Anwendung von Mischungsvariante b) gegebenenfalls uncalcinierte) Trockengemisch aber auch als Pulverkatalysator eingesetzt werden.

Eigene Untersuchungen haben ergeben, daß beim Calcinieren des die Ausgangsmasse 1 und die Ausgangsmasse 2 umfassenden Trockengemisches der Strukturtyp der in der Ausgangsmasse 1 enthaltenen Kristallite B* als solcher im wesentlichen erhalten bleibt. Enthält die Ausgangsmasse 1 noch andere Strukturtypen der Tabelle 1, so bleiben diese bei der Calcination des Trockengemisches entweder als solche ebenfalls enthalten (insbesondere im Falle von Kristalliten von Oxometallaten III des Wolframit-Strukturtyps) oder wandeln sich teilweise oder vollständig in andere Strukturtypen der Tabelle 1 um. Ein Verschmelzen (Ineinanderlösen) der Bestandteile der Ausgangsmasse 1 mit jenen der Ausgangsmasse 2 findet jedoch im wesentlichen nicht statt.

Dies eröffnet die Möglichkeit, nach Mahlen des vorgebildeten Ausgangsgemisches 1, bzw. Ausgangsgemisches 1' bzw. Ausgangsgemisches 1'' (z.B. durch Naß- oder Trockenmahlen, z.B. in der Kugelmühle oder durch Strahlmahlen) aus dem dabei erhältlichen, in der Regel aus im wesentlichen kugelförmigen Partikeln bestehenden Pulver, die Kornklasse mit einem im für die Multimetalloxidmasse I gewünschten Größtdurchmesserbereich liegenden Korngrößtdurchmesser (in der Regel > 0 bis 300 µm, vorzugsweise 0,1 bis 200 µm, besonders bevorzugt 0,5 bis 50 µm und ganz besonders bevorzugt 1 bis 30 µm) durch in an sich bekannter Weise durchzuführendes Klassieren (z.B. Naß- oder Trockensiebung) abzutrennen und so zur Herstellung der gewünschten Multimetalloxidmasse maßgeschneidert einzusetzen.

Bemerkenswerterweise sind solche erfindungsgemäßen Multimetalloxidmassen I als Katalysatoren für die gasphasenkatalytische Oxidation von Acrolein zu Acrylsäure besonders vorteilhaft, deren Promotorphase B sowohl Oxometallate II des HT-Cu-Molybdat-Strukturtyps (d.h. Kristallite B*) als auch Kristallite von Oxometallaten III des Wolframit-Strukturtyps (hier als Kristallite B** bezeichnet) enthalten. Das Gewichtsverhältnis von in der Promotorphase B enthaltenen Kristalliten B* zu Kristalliten B** kann dabei 0,01 bis 100, 0,1 bis 10, 0,25 bis 4 sowie 0,5 bis 2 betragen. Dies gilt insbesondere dann, wenn die Promotorphase B der erfindungsgemäßen Multimetalloxidmassen I ausschließlich aus einem Gemisch von solchen Kristalliten B* und Kristalliten B** besteht, wobei es von Vorteil ist, wenn die Kristallite B* mit den Kristalliten B** verwachsen sind.

Bevorzugt sind dabei Gemische von Kristalliten B* der allgemeinen Formel V und Kristalliten B** der Stöchiometrie VI

$$CuMo_{A'}W_{B'}V_{C'}O_{Y'} \qquad (VI),$$

mit

| | |
|---|---|
| 1/(A'+B'+C') | 0,7 bis 1,3, |
| A',B',C' | alle >0 mit der Maßgabe, daß B'+C' ≤ 1 und |
| Y' | eine Zahl, die durch die Wertigkeit und Häufigkeit der von Sauerstoff verschiedenen Elemente in VI bestimmt wird, |

und/oder der Stöchiometrie VII

$$CuMo_{A'}W_{B'}O_{Y'} \qquad (VII),$$

mit

| | |
|---|---|
| 1/(A'+B') | 0,7 bis 1,3, |
| B'/A' | 0,01 bis 1 und |
| Y' | eine Zahl, die durch die Wertigkeit und Häufigkeit der von Sauerstoff verschiedenen Elemente in VII bestimmt wird. |

Interessanterweise entstehen solche verwachsenen Gemische B*/B** häufig bei der Calcination von innigen Gemischen geeigneter Quellen ihrer elementaren Konstituenten, wenn die Gemische diese elementaren Konstituenten in der Stöchiometrie III enthalten und wenn die Calcination unter Inertgas oder an Luft sowie bei 700 bis 900°C erfolgt.

Bei Verwendung der erfindungsgemäßen Multimetalloxidmassen als Katalysatoren für die gasphasenkatalytische Oxidation von Acrolein zu Acrylsäure erfolgt die Formgebung zur gewünschten Katalysatorgeometrie vorzugsweise durch Aufbringen auf vorgeformte inerte Katalysatorträger, wobei das Aufbringen vor oder nach der abschließenden Calcination erfolgen kann. Dabei können die üblichen Trägermaterialien wie poröse oder unporöse Aluminiumoxide, Siliciumdioxid, Thoriumdioxid, Zirkondioxid, Siliciumcarbid oder Silicate wie Magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln besonders vorteilhaft. Von besonderem Vorteil ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Die Schichtdicke der Aktivmasse wird zweckmäßigerweise als im Bereich 50 bis 500 µm, bevorzugt im Bereich 150 bis 250 µm liegend, gewählt. Es sei an dieser Stelle darauf hingewiesen, daß bei der Herstellung solcher Schalenkatalysatoren zur Beschichtung der Trägerkörper die aufzubringende Pulvermasse in der Regel befeuchtet und nach dem Aufbringen, z.B. mittels heißer Luft, wieder getrocknet wird.

Die Beschichtung der Trägerkörper wird zur Herstellung der Schalenkatalysatoren in der Regel in einem geeigneten drehbaren Behälter ausgeführt, wie er z.B. aus der DE-A 2909671 oder aus der EP-A 293859 vorbekannt ist. In der Regel wird die relevante Masse vor der Trägerbeschichtung calciniert.

In geeigneter Weise kann das Beschichtungs- und Calcinierungsverfahren gemäß der EP-A 293 859 in an sich bekannter Weise so angewendet werden, daß die resultierenden Multimetalloxidaktivmassen eine spezifische Oberfläche von 0,50 bis 150 $m^2/g$, ein spezifisches Porenvolumen von 0,10 bis 0,90 $cm^3/g$ und eine solche Porendurchmesser-Verteilung aufweisen, daß auf die Durchmesserbereiche 0,1 bis < 1 μm, 1,0 bis < 10 μm und 10 μm bis 100 μm jeweils wenigstens 10 % des Porengesamtvolumens entfallen. Auch können die in der EP-A 293 859 als bevorzugt genannten Porendurchmesser-Verteilungen eingestellt werden.

Selbstverständlich können die erfindungsgemäßen Multimetalloxidmassen auch als Vollkatalysatoren betrieben werden. Diesbezüglich wird das die Ausgangsmasse 1 und 2 umfassende innige Trockengemisch vorzugsweise unmittelbar zur gewünschten Katalysatorgeometrie verdichtet (z.B. Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und calciniert. Generell kann auch hier vor der Formgebung calciniert werden. Bevorzugte Vollkatalysatorgeometrie sind Hohlzylinder mit einem Außendurchmesser und einer Länge von 2 bis 10 mm und einer Wandstärke von 1 bis 3 mm.

Die erfindungsgemäßen Multimetalloxidmassen eignen sich insbesondere als Katalysatoren mit erhöhter Selektivität (bei vorgegebenem Umsatz) für die gasphasenkatalytische Oxidation von Acrolein zu Acrylsäure. Normalerweise wird bei dem Verfahren Acrolein eingesetzt, das durch die katalytische Gasphasenoxidation von Propen erzeugt wurde. In der Regel werden die Acrolein enthaltenden Reaktionsgase dieser Propenoxidation ohne Zwischenreinigung eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt (z.B. in Form von Luft), eingesetzt. Geeignete Verdünnungsgase sind z.B. $N_2$, $CO_2$, Kohlenwasserstoff, rückgeführte Reaktionsabgase und/oder Wasserdampf. In der Regel wird bei der Acroleinoxidation ein Acrolein:Sauerstoff:Wasserdampf:Inertgas-Volumenverhältnis von 1:(1 bis 3):(0 bis 20):(3 bis 30) vorzugsweise von 1:(1 bis 3):(0,5 bis 10):(7 bis 18) eingestellt. Der Reaktionsdruck beträgt im allgemeinen 1 bis 3 bar und die Gesamtraumbelastung beträgt vorzugsweise 1000 bis 3500 Nl/l/h. Typische Vielrohr-Festbettreaktoren sind z.B. in den Schriften DE-A 2830765, DE-A 2 201 528 oder US-A 3 147 084 beschrieben. Die Reaktionstemperatur wird üblicherweise so gewählt, daß der Acroleinumsatz bei einfachem Durchgang oberhalb von 90 %, vorzugsweise oberhalb von 98 %, liegt. Im Normalfall sind diesbezüglich Reaktionstemperaturen von 230 bis 330°C erforderlich.

Bemerkenswerterweise weisen die erfindungsgemäßen Multimetalloxidmassen im Rahmen der gasphasenkatalytischen Oxidation von Acrolein zu Acrylsäure bezüglich der Selektivität der Acrylsäurebildung auch eine reduzierte Formierungszeit auf; d.h. wird ein mit den erfindungsgemäßen Multimetalloxidmassen beschickter Rohrbündelreaktor unter den vorgenannten Bedingungen mit einem Acrolein enthaltenden Gasstrom zum Zwecke der oxidativen Bildung von Acrylsäure betrieben, so erreicht die Selektivität der Acrylsäurebildung bereits innerhalb einer reduzierten Betriebsdauer ihren Plateauwert. Bezüglich dieses Plateauwertes verfügt die Herstellung der erfindungsgemäßen Multimetalloxidmassen darüberhinaus über eine erhöhte Reproduzierbarkeit.

Neben der gasphasenkatalytischen Oxidation von Acrolein zu Acrylsäure vermögen die erfindungsgemäßen Verfahrensprodukte aber auch die gasphasenkatalytische Oxidation anderer organischer Verbindungen wie insbesondere anderer, vorzugsweise 3 bis 6 C-Atome aufweisender, Alkane, Alkanole, Alkanale, Alkene und Alkenole (z.B. Propylen, Methacrolein tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung olefinischer Verbindungen.

Abschließend sei festgehalten, daß sowohl die Kristallite B* als auch die Kristallite B** in völlig analoger Weise wie vorstehend beschrieben, auch in den in der DE-A 44 05 058 und DE-A 44 05 060 beanspruchten und beschriebenen Multimetalloxidmassen als Schlüsselphasen eingesetzt und die dabei resultierende Multimetalloxidmassen als Gasphasenoxidationskatalysatoren, wie in den beiden DE-As beschrieben, verwendet werden können.

Umsatz, Selektivität und Verweilzeit sind in dieser Schrift, falls nichts anderes erwähnt wird, wie folgt definiert:

$$\text{Umsatz U an Acrolein (\%)} = \frac{\text{Molzahl umgesetztes Acrolein}}{\text{Molzahl eingesetztes Acrolein}} \times 100;$$

$$\text{Selektivität S der Acrylsäurebildung \%} = \frac{\text{Molzahl Acrolein umgesetzt zu Acrylsäure}}{\text{Molzahl Acrolein insgesamt umgesetzt}} \times 100;$$

$$\text{Verweilzeit (sec)} = \frac{\text{mit Katalysator gefülltes Leervolumen des Reaktors (l)}}{\text{durchgesetzte Synthesegasmenge (Nl/h)}} \times 3600;$$

Beispiele

a) Herstellung von erfindungsgemäßen Multimetalloxidmassen M und Multimetalloxidmassen MV zum Vergleich

MV1: 127 g Kupfer(II)acetatmonohydrat (Cu-Gehalt: 32,4 Gew.-%) wurden in 2700 g Wasser zu einer Lösung I gelöst. In 5500 g Wasser wurden bei 95°C nacheinander 860 g Ammoniumheptamolybdattetrahydrat (81,3 Gew.-% $MoO_3$), 143 g Ammoniummetavanadat (72,2 Gew.-% $V_2O_5$) und 126 g Ammoniumparawolframatheptahydrat (89,3 Gew.-% $WO_3$) zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet. Die Knetmasse wurde in einem mit einem Sauerstoff/Stickstoff-Gemisch beschickten Umluftofen calciniert. Der Sauerstoffgehalt wurde dabei so eingestellt, daß am Ausgang des Umluftofens ein $O_2$-Gehalt von 1,5 Vol.-% bestand. Im Rahmen der Calcinierung wurde die Knetmasse zunächst mit einer Geschwindigkeit von 10°C/min auf 300°C aufgeheizt und anschließend während 6 h auf dieser Temperatur gehalten. Danach wurde mit einer Geschwindigkeit von 10°C/min auf 400°C aufgeheizt und diese Temperatur noch 1 h aufrechterhalten. Zur Einstellung des Ammoniakgehaltes der Calcinierungsatmosphäre wurden die Ofenbeladung O (g Katalysatorvorläufer pro l Innenvolumen des Umluftofens), der Eingangsvolumenstrom ES (Nl/h) des Sauerstoff/Stickstoff-Gemisches und die Verweilzeit VZ (sec) der Sauerstoff/Stickstoff-Beschickung (Verhältnis aus Innenvolumen des Umluftofens und Volumenstrom des zugeführten Sauerstoff/Stickstoff-Gemisches) wie nachfolgend aufgelistet gewählt. Der verwendete Umluftofen wies ein Innenvolumen von 3 l auf.
O: 250 g/l,
VZ: 135 sec und
ES: 80 Nl/h.
Dem resultierenden katalytisch aktiven Material liegt folgende Stöchiometrie zugrunde:
$Mo_{12}V_3W_{1,2}Cu_{1,6}O_x$.
Nach Mahlen des calcinierten, katalytisch aktiven Materials auf Teilchendurchmesser im Bereich von 0,1 bis 50 µm wurden mit dem dabei resultierenden Aktivmassenpulver in einer Drehtrommel unporöse, oberflächenrauhe Steatitkugeln eines Durchmessers von 4 bis 5 mm in einer Menge von 50 g Pulver je 200 g Steatitkugeln bei gleichzeitigem Zusatz von 18 g Wasser beschichtet. Anschließend wurde mit 110°C heißer Luft getrocknet.

M1: Ausgangsmasse 1:
371,76 g Ammoniumheptamolybdathydrat ($MoO_3$-Gehalt: 81,3 Gew.-%, Idealzusammensetzung: $(NH_4)_6Mo_7O_{24} \times 4H_2O$) und 60,82 g Ammoniumparawolframathydrat ($WO_3$-Gehalt: 89,2 Gew.-%, Idealzusammensetzung: $(NH_4)_{10}W_{12}O_{41} \times 7H_2O$) wurden bei 90°C in 5 l Wasser unter Rühren gelöst (Lösung A). 455,68 g Kupferacetathydrat (Cu-Gehalt: 32,5 Gew.-%, Idealzusammensetzung: $Cu(CH_3COO)_2 \times H_2O$) wurden mit 3 l Wasser und 181,36 g einer 25 gew.-%igen wäßrigen Ammoniaklösung versetzt und bei 25°C 15 min gerührt, wobei eine hellblaue wäßrige Suspension erhalten wurde (Suspension B). Anschließend wurde die Suspension B in die 90°C aufweisende Lösung A eingerührt und die dabei resultierende wäßrige Suspension C 3 h bei 80°C gerührt. Nach Abkühlen der resultierenden wäßrigen Suspension C auf 25°C wies deren wäßriges Suspendiermedium einen pH-Wert von 5,0 auf (Glaselektrode). Anschließend wurde die Suspension C bei einer Eingangstemperatur von 310°C und einer Ausgangstemperatur von 110°C sprühgetrocknet. Das dabei anfallende hellgrüne Pulver wurde an Luft calciniert, wobei in einem ersten Schritt innerhalb von 24 h kontinuierlich von 25°C auf 300°C und in einem sich daran anschließenden zweiten Schritt innerhalb von 3 h kontinuierlich von 300°C auf 780°C erhitzt sowie in einem dritten Schritt die Temperatur noch 1 h bei 780°C gehalten wurde.
Die Calcination wurde in einem Drehkugelofen mit einem nutzbaren Volumen von 1 l durchgeführt, wobei jeweils 60 g Ausgangssprühpulver eingesetzt und ein Luftstrom von 50 Nl/h eingestellt wurde.
Das resultierende Pulver wies eine braune Farbe und eine spezifische Oberfläche (bestimmt nach DIN 66131 durch Gasadsorption ($N_2$) gemäß Brunauer-Emmet-Teller (BET)) von 0,3 $m^2$/g sowie die Zusammensetzung $CuMo_{0,9}W_{0,1}O_{3,5-4}$ auf. Bei der rasterelektronenmikroskopischen Untersuchung (REM) wies das Pulver kristalline Partikel mit einem zahlenmittleren Korngrößtdurchmesser von etwa 8 µm aus. Unter Anwendung von Cu-Kα-Strahlung (Siemens-Diffraktometer D-5000, 40 kV, 30 mA, mit automatischer Divergenz-, Streustrahl- und Zählrohrblende sowie Peltier-Detektor) zeigte das kristalline Pulver $CuMo_{0,9}W_{0,1}O_{3,5-4}$ das nachfolgende Röntgenbeugungsmuster, wiedergegeben in Gestalt von von der Wellenlänge der verwendeten Röntgenstrahlung unabhängigen Netzebenenabständen d[Å], sowie den zugehörigen, auf die intensitätsstärkste Beugungslinie bezogenen, relativen Intensitäten (%) der verschiedenen Beugungslinien (angeordnet nach abnehmender Intensität):

| d [Å] | Intensität [%] |
|---|---|
| 3,40 | 100 |
| 3,54 | 72 |
| 2,27 | 39 |
| 6,79 | 32 |
| 2,56 | 25 |
| 1,57 | 22 |
| 1,87 | 19 |
| 2,96 | 18 |
| 3,56 | 18 |
| 1,64 | 17 |
| 2,66 | 16 |
| 1,59 | 16 |
| 1,55 | 16 |
| 2,67 | 14 |
| 2,00 | 14 |
| 3,04 | 13 |
| 1,57 | 11 |
| 2,36 | 11 |
| 1,44 | 11 |
| 1,70 | 10 |
| 1,51 | 10 |
| 2,35 | 10 |
| 1,55 | 9,6 |
| 2,32 | 9,2 |
| 2,89 | 9,0 |
| 1,68 | 9,0 |
| 1,48 | 8,9 |
| 1,94 | 8,7 |
| 1,60 | 8,7 |
| 2,69 | 8,6 |

| d [Å] | Intensität [%] |
|---|---|
| 1,84 | 8,5 |

(fortgesetzt)

| d [Å] | Intensität [%] |
|---|---|
| 1,99 | 8,1 |
| 3,92 | 8,0 |
| 2,34 | 8,0 |
| 2,70 | 7,5 |
| 1,52 | 7,5 |
| 1,49 | 7,4 |
| 2,44 | 7,3 |
| 5,78 | 7,2 |
| 1,68 | 7,1 |
| 1,91 | 6,9 |
| 1,71 | 6,8 |
| 1,74 | 6,5 |
| 4,56 | 6,3 |
| 3,16 | 6,1 |
| 2,08 | 5,7 |
| 2,02 | 5,6 |
| 2,28 | 5,6 |
| 2,05 | 5,5 |
| 1,80 | 5,4 |
| 5,13 | 4,9 |
| 3,48 | 4,9 |
| 3,12 | 4,1 |
| 4,20 | 3,7 |
| 4,39 | 3,5 |
| 3,84 | 3,5 |
| 3,73 | 3,4 |
| 4,68 | 3,4 |
| 4,46 | 3,2 |
| 3,76 | 2,9 |

Die Ungenauigkeit der Angabe der Netznebenenabstände d beläuft sich für d-Werte ≥ 2,9 Å im wesentlichen auf ± 0,3 Å und für d-Werte < 2,9 Å im wesentlichen auf ± 0,2 Å (die intensitätsarmen Linien umfassen eventuell auch auf geringfügige Verunreinigungen zurückgehende Linien).

Ausgangsmasse 2:

In 5500 g Wasser wurden bei 95°C nacheinander 777,7 g Ammoniumheptamolybdattetrahydrat (81,3 Gew.-% $MoO_3$), 147,0 g Ammoniummetavanadat (77,2 Gew.-% $V_2O_5$) und 112,1 g Ammoniumparawolframatheptahydrat (89,3 Gew.-% $WO_3$) gelöst. Der wäßrigen Lösung (Ausgangsmasse 2) lag somit nachfolgende Elementstöchiometrie zugrunde:

$$Mo_{12}V_{3,41}W_{1,18}.$$

Von der Ausgangsmasse 1 wurde nach Mahlen in einer Zentrifugalmühle der Fa. Retsch, DE, auf einen zahlenmittleren Korngrößtdurchmesser von 1 bis 3 µm soviel in die Ausgangsmasse 2 eingerührt, daß das molare Verhältnis der vorgenannten stöchiometrischen Einheiten 1,6 (Ausgangsmasse 1) zu 0,88 (Ausgangsmasse 2) betrug. Anschließend wurden in die wäßrige Mischung noch 102,5 g Ammoniumacetat eingerührt, die resultierende Suspension bei 95°C 1 h nachgerührt und danach auf 80°C abgekühlt. Anschließend wurde die wäßrige Mischung wie in MV1 sprühgetrocknet und zu einem Schalenkatalysator weiterverarbeitet, dessen Aktivmasse somit ebenfalls die Bruttostöchiometrie $Mo_{12}V_3W_{1,2}Cu_{1,6}O_x$

$$\triangleq \left[Mo_{12}V_{3,41}W_{1,18}O_{x'}\right]_{0,88}\left[CuMo_{0,9}W_{0,1}O_{3,5-4}\right]_{1,6}$$

aufwies. Das Röntgenspektrum der Aktivmasse enthielt nach wie vor den HT-Cu-Molybdattyp.

MV2: Ausgangsmasse 1:

263,83 g Ammoniumheptamolybdathydrat ($MoO_3$-Gehalt: 81,8 Gew.-%, Idealzusammensetzung: $(NH_4)_6Mo_7O_{24}x4H_2O$) und 260,26 g Ammoniumparawolframathydrat ($WO_3$-Gehalt: 89,0 Gew.-%, Idealzusammensetzung: $(NH_4)_{10}W_{12}O_{41}x7H_2O$) wurden bei 90°C in 4 l Wasser gelöst (Lösung A). 496,37 g Kupferacetathydrat (Cu-Gehalt: 32,0 Gew.-%, Idealzusammensetzung: $Cu(CH_3COO)_2xH_2O$) wurden mit 4 l Wasser und 435 g einer 25 gew.-%igen wäßrigen Ammoniaklösung versetzt und bei 25°C 15 min gerührt, wobei eine blaue Lösung erhalten wurde (Lösung B). Anschließend wurde die Lösung B in die 90°C aufweisende Lösung A eingerührt und die resultierende Mischung noch 3 h bei 70°C nachgerührt. Nach Abkühlen der resultierenden wäßrigen Mischung C auf 25°C wies deren wäßriges Medium einen pH-Wert von 8,4 auf (Glaselektrode). Anschließend wurde die Mischung C bei einer Eingangstemperatur von 330°C und einer Ausgangstemperatur von 110°C sprühgetrocknet. Das dabei anfallende grüne Pulver wurde an Luft calciniert, wobei in einem ersten Schritt innerhalb von 24 h kontinuierlich von 25°C auf 300°C und in einem sich daran anschließenden zweiten Schritt innerhalb von 12 h kontinuierlich von 300 auf 400°C erhitzt sowie in einem dritten Schritt die Temperatur noch 1 h bei 400°C gehalten wurde. Im übrigen wurde die Calcination wie bei Ausgangsmasse 1 für M1 durchgeführt.

Das resultierende Pulver wies eine braune Farbe und eine spezifische Oberfläche nach DIN 66131 von 9,5 $m^2/g$ sowie die Zusammensetzung $CuMo_{0,6}W_{0,4}O_4$ auf. Bei der REM-Untersuchung wies das Pulver kristalline Partikel enthaltende Kugeln mit einem zahlenmittleren Partikelgrößtdurchmesser von etwa 0,2 µm aus.

Unter Anwendung von Cu-Kα-Strahlung (Siemens-Diffraktometer D-5000, 40 kV, 30 mA, mit automatischer Divergenz-, Streustrahl- und Zählrohrblende sowie Peltier-Detektor) zeigte das kristalline Pulver ein Pulver-Röntgendiagramm, das dem Wolframit-Strukturtyp ($CuMoO_4$-III gemäß Russian Journal of Inorganic Chemistry 36(7) (1991) S. 927 Table 1 bzw. $CuWO_4$ gemäß der Karteikarte 21-307 der JCPDS-ICDD-Kartei (1994)) entsprach.

Eine weitere, über die Wolframit-Struktur hinausgehende Phase war nicht vorhanden.

Ausgangsmasse 2:

Eine wie bei M1 erzeugte wäßrige Lösung, die zugrunde liegende Elementstöchiometrie war jedoch

$$Mo_{12}V_{3,26}W_{0,61}.$$

Von der Ausgangsmasse 1 wurde nach Mahlen gemäß Ausgangsmasse 1 aus M1 soviel in die Ausgangsmasse 2 eingerührt, daß das molare Verhältnis der vorgenannten stöchiometrischen Einheiten 1,6 (Ausgangsmasse 1) zu 0,92 (Ausgangsmasse 2) betrug.

Anschließend wurde die wäßrige Mischung wie in M1 behandelt, sprühgetrocknet und zu einem Schalenkatalysator weiterverarbeitet, dessen Aktivmasse somit ebenfalls die Bruttostöchiometrie $Mo_{12}V_3W_{1,2}Cu_{1,6}O_x$

$$\triangleq \left[Mo_{12}V_{3,26}W_{0,61}O_{x'}\right]_{0,92}\left[CuMo_{0,6}W_{0,4}O_4\right]_{1,6}$$

aufwies. Das Röntgenspektrum der Aktivmasse enthielt nach wie vor den Wolframit-Typ.

M2: Ausgangsmasse 1:

Wie zur Herstellung der Ausgangsmasse 1 für MV2 wurde ein Sprühtrocknungspulver hergestellt, das die Elemente Cu, Mo und W im Molverhältnis 1:0,6:0,4 enthielt. Das dabei erhaltene grüne Pulver wurde an Luft calciniert, wobei in einem ersten Schritt innerhalb von 24 h kontinuierlich von 25°C auf 300°C und in einem sich daran anschließenden zweiten Schritt innerhalb von 3 h kontinuierlich von 300°C auf 700°C erhitzt, sowie in einem dritten Schritt die Temperatur noch 1 h bei 700°C gehalten wurde. Im übrigen wurde die Calcination wie bei Ausgangsmasse 1 für M1 durchgeführt.

Das resultierende Pulver wies eine braune Farbe und eine spezifische Oberfläche nach DIN 66131 von 0,8 $m^2/g$ sowie die Zusammensetzung $CuMo_{0,6}W_{0,4}O_{3,5-4}$ auf. Bei der REM-Untersuchung wies das Pulver kristalline Partikel mit einem zahlenmittleren Korngrößtdurchmesser von etwa 8 µm aus. Unter Anwendung von Cu-Kα-Strahlung (Siemens-Diffraktometer D-5000, 40 kV, 30 mA, mit automatischer Divergenz-, Streustrahl- und Zählrohrblende sowie Peltier-Detektor) zeigte das kristalline Pulver der Zusammensetzung $CuMo_{0,6}W_{0,4}O_{3,5-4}$ ein Pulver-Röntgendiagram, das eine Superposition des Wolframit-Fingerabdrucks mit dem HT-Cu-Molybdat-Fingerabdrucks zeigte, d.h. es besaß einen zweiphasigen Aufbau.

Gemäß den Linienintensitäten lagen beide Strukturtypen in etwa derselben Häufigkeit (ca. 50 Gew.-%) vor.

Ausgangsmasse 2:

Eine wie bei M1 erzeugte wäßrige Lösung, die zugrunde liegende Elementstöchiometrie war jedoch

$Mo_{12}V_{3,26}W_{0,61}$.

Von der Ausgangsmasse 1 wurde nach Mahlen gemäß Ausgangsmasse 1 aus M1 soviel in die Ausgangsmasse 2 eingerührt, daß das molare Verhältnis der vorgenannten stöchiometrischen Einheiten 1,6 (Ausgangsmasse 1) zu 0,92 (Ausgangsmasse 2) betrug. Anschließend wurde die wäßrige Mischung wie in M1 behandelt, sprühgetrocknet und zu einem Schalenkatalysator weiterverarbeitet, dessen Aktivmasse somit ebenfalls die Bruttostöchiometrie $Mo_{12}V_3W_{1,2}Cu_{1,6}O_x$

$$\hat{=} \left[ Mo_{12}V_{3,26}W_{0,61}O_x, \right]_{0,92} \left[ CuMo_{0,6}W_{0,4}O_{3,5-4} \right]_{1,6}$$

aufwies.

Das Röntgenspektrum der Aktivmasse enthielt nach wie vor die Superposition von HT-Cu-Molybdat- und Wolframit-Typ.

MV3: Ausgangsmasse 1:

223,05 g Ammoniumheptamolybdathydrat ($MoO_3$-Gehalt: 81,3 Gew.-%, Idealzusammensetzung: $(NH_4)_6Mo_7O_{24}x4H_2O$) und 327,52 g Ammoniumparawolframathydrat ($WO_3$-Gehalt: 89,2 Gew.-%, Idealzusammensetzung: $(NH_4)_{10}W_{12}O_{41}x7H_2O$) wurden bei 90°C in 5 l Wasser unter Rühren gelöst (Lösung A). 492,64 g Kupferacetathydrat (Cu-Gehalt: 32,5 Gew.-%, Idealzusammensetzung: $Cu(CH_3COO)_2xH_2O$) wurden mit 3 l Wasser und 197,88 g einer 25 gew.-%igen wäßrigen Ammoniaklösung versetzt und 15 min bei 25°C gerührt, wobei eine hellblaue Suspension erhalten wurde (Suspension B). Anschließend wurde die Suspension B in die 90°C aufweisende Lösung A eingerührt und die dabei resultierende Suspension 3 h bei 80°C nachgerührt. Das wäßrige Suspendiermedium der resultierenden wäßrigen Suspension (Suspension C) wies nach Abkühlen auf 25°C einen pH-Wert (Glaselektrode) von 5,3 auf. Die Suspension C wurde bei einer Eingangstemperatur von 310°C und einer Austrittstemperatur von 110°C sprühgetrocknet. Das anfallende hellgrüne Pulver wurde an Luft calciniert, wobei in einem ersten Schritt innerhalb von 24 h kontinuierlich von 25°C auf 300°C und in einem sich daran anschließenden zweiten Schritt innerhalb von 1 h kontinuierlich von 300°C auf 400°C erhitzt, sowie in einem dritten Schritt die Temperatur noch 1 h bei 400°C gehalten wurde. Im übrigen wurde die Calcination wie bei Ausgangsmasse 1 für M1 durchgeführt.

Das resultierende Pulver wies eine braune Farbe und eine spezifische Oberfläche nach DIN 66131 von 12,2 $m^2/g$ sowie die Zusammensetzung $CuMo_{0,5}W_{0,5}O_4$ auf. Bei der REM-Untersuchung wies das Pulver kristalline Partikel enthaltende Kugeln mit einem zahlenmittleren Partikelgrößtdurchmesser von etwa 0,15 µm aus. Unter Anwendung von Cu-Kα-Strahlung (Siemens-Diffraktometer D-5000,40 kV, 30 mA, mit automatischer Divergenz-, Streustrahl- und Zählrohrblende sowie Peltier-Detektor) zeigte das kristalline Pulver der Zusammensetzung $CuMo_{0,5}W_{0,5}O_4$ ein Pulver-Röntgendiagramm, das dem Wolframit-Strukturtyp entsprach. Eine weitere, über die Wolframit-Struktur hinausgehende Phase war nicht vorhanden.

Ausgangsmasse 2:

Eine wie bei M1 erzeugte wäßrige Lösung, die zugrunde liegende Elementstöchiometrie war jedoch

$$Mo_{12}V_{3,21}W_{0,43.}$$

Von der Ausgangsmasse 1 wurde soviel in die Ausgangsmasse 2 eingerührt, daß das molare Verhältnis der vorgenannten stöchiometrischen Einheiten 1,6 (Ausgangsmasse 1) zu 0,93 (Ausgangsmasse 2) betrug.
Anschließend wurde die wäßrige Mischung wie in M1 behandelt, sprühgetrocknet und zu einem Schalenkatalysator weiterverarbeitet, dessen Aktivmasse somit ebenfalls die Bruttostöchiometrie $Mo_{12}V_3W_{1,2}Cu_{1,6}O_x$

$$\triangleq \left[ Mo_{12}V_{3,21}W_{0,43}O_x, \right]_{0,93} \left[ CuMo_{0,5}W_{0,5}O_4 \right]_{1,6}$$

aufwies.
Das Röntgenspektrum der Aktivmasse enthielt nach wie vor den Wolframit-Typ.

M3: Ausgangsmasse 1:
Wie zur Herstellung der Ausgangsmasse 1 für MV3 wurde ein Sprühtrocknungspulver hergestellt, das die Elemente Cu, Mo und W im Molverhältnis 1:0,5:0,5 enthielt. Das dabei erhaltene grüne Pulver wurde an Luft calciniert, wobei in einem ersten Schritt innerhalb von 24 h kontinuierlich von 25°C auf 300°C und in einem sich daran anschließenden zweiten Schritt innerhalb von 3 h kontinuierlich von 300 auf 780°C erhitzt, sowie in einem dritten Schritt die Temperatur noch 1 h bei 780°C gehalten wurde. Im übrigen wurde die Calcination wie bei Ausgangsmasse 1 für M1 durchgeführt.
Das resultierende Pulver wies eine braune Farbe und eine spezifische Oberfläche nach DIN 66131 von 0,3 $m^2/g$ sowie die Zusammensetzung $CuMo_{0,5}W_{0,5}O_{3,5\text{-}4}$ auf. Bei der REM-Untersuchung wies das Pulver kristalline Partikel mit einem zahlenmittleren Korngrößtdurchmesser von etwa 8 μm aus. Unter Anwendung von Cu-Kα-Strahlung (Siemens-Diffraktometer D-5000, 40 kV, 30 mA, mit automatischer Divergenz-Streustrahl- und Zählrohrblende sowie Peltier-Detektor) zeigte das kristalline Pulver der Zusammensetzung $CuMo_{0,5}W_{0,5}O_{3,5\text{-}4}$ ein Pulver-Röntgendiagramm, das eine Superposition des Wolframit-Fingerabdrucks mit dem HT-Cu-Molybdat-Fingerabdruck zeigte, d.h. es besaß einen zweiphasigen Aufbau. Gemäß den Linienintensitäten lagen die beiden Strukturtypen etwa im Häufigkeitsverhältnis 60 (Wolframit-Struktur): 40 (HT-Cu-Molybdat-Typ) vor.
Ausgangsmasse 2:
Eine wie bei M1 erzeugte wäßrige Lösung, die zugrunde liegende Elementstöchiometrie war jedoch

$$Mo_{12}V_{3,21}W_{0,43.}$$

Von der Ausgangsmasse 1 wurde nach Mahlen gemäß Ausgangsmasse 1 aus M1 soviel in die Ausgangsmasse 2 eingerührt, daß das molare Verhältnis der vorgenannten stöchiometrischen Einheiten 1,6 (Ausgangsmasse 1) zu 0,93 (Ausgangsmasse 2) betrug.
Anschließend wurde die wäßrige Mischung wie in M1 behandelt, sprühgetrocknet und zu einem Schalenkatalysator weiterverarbeitet, dessen Aktivmasse somit ebenfalls die Bruttostöchiometrie $Mo_{12}V_3W_{1,2}Cu_{1,6}O_x$

$$\triangleq \left[ Mo_{12}V_{3,21}W_{0,43}O_x, \right]_{0,93} \left[ CuMo_{0,5}W_{0,5}O_{3,5\text{-}4} \right]_{1,6}$$

aufwies.
Das Röntgenspektrum der Aktivmasse enthielt nach wie vor die Superposition von Wolframit-Typ und HT-Cu-Molybdat-Typ.

b. Verwendung der Schalenkatalysatoren aus a. als Katalysatoren für die Gasphasenoxidation von Acrolein zu Acrylsäure

Die Katalysatoren wurden in einen Rohrreaktor gefüllt (V2A-Stahl, 25 mm Innendurchmesser, 2000 g Katalysatorschüttung, Salzbadtemperierung) und bei Reaktionstemperaturen im Bereich von 250 bis 270°C unter Anwendung einer Verweilzeit von 2,0 sec mit einem gasförmigen Gemisch der Zusammensetzung

| | |
|---|---|
| 5 Vol.-% | Acrolein, |
| 7 Vol.-% | Sauerstoff, |
| 10 Vol.-% | Wasserdampf und |
| 78 Vol.-% | Stickstoff |

beschickt. Die Salzbadtemperatur wurde in allen Fällen so eingestellt, daß, nach beendeter Formierung, bei einfachem Durchgang ein einheitlicher Acroleinumsatz U von 99 % resultierte. Das aus dem Reaktor strömende Produktgasgemisch wurde gaschromatographisch analysiert. Die Ergebnisse für die Selektivität der Acrylsäurebildung in Anwendung der verschiedenen Katalysatoren zeigt die nachfolgende Tabelle.

| Katalysator | S (%) |
|---|---|
| MV1 | 95,3 |
| M1 | 95,6 |
| MV2 | 95,7 |
| M2 | 95,9 |
| MV3 | 96,1 |
| M3 | 96,5 |

## Patentansprüche

**1.** Multimetalloxidmassen der allgemeinen Formel I

$$[A]_p\ [B]_q \qquad (I),$$

in der die Variablen folgende Bedeutung haben:

A $Mo_{12}\ V_a\ X^1_b\ X^2_c\ X^3_d\ X^4_e\ X^5_f\ X^6_g\ O_x$ (Aktivphase),

B $X^7_{12}Cu_h\ H_i\ O_y$ (Promotorphase),

$X^1$ W, Nb, Ta, Cr und/oder Ce,

$X^2$ Cu, Ni, Co, Fe, Mn und/oder Zn,

$X^3$ Sb und/oder Bi,

$X^4$ Li, Na, K, Rb, Cs und/oder H,

$X^5$ Mg, Ca, Sr und/oder Ba,

$X^6$ Si, Al, Ti und/oder Zr,

$X^7$ Mo, W, V, Nb und/oder Ta,

a 1 bis 8,

b 0,2 bis 5,

c 0 bis 23,

d 0 bis 50,

e 0 bis 2,

f 0 bis 5,

g 0 bis 50,

h 4 bis 30,

i 0 bis 20,

x,y Zahlen, die durch die Wertigkeit und Häufigkeit der von Sauerstoff verschiedenen Elemente in I bestimmt werden und

p,q von Null verschiedene Zahlen, deren Verhältnis p/q 160:1 bis 1:1 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A $Mo_{12} V_a X^1_b X^2_c X^3_d X^4_e X^5_f X^6_g O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B $X^7_{12} Cu_h H_i O_y$

enthalten, wobei die Bereiche A, B relativ zueinander wie in einem Gemisch aus feinteiligem A und feinteiligem B verteilt sind, mit der Maßgabe, daß die Bereiche B Kristallite B* von Oxometallaten der allgemeinen Formel II

$$Cu\ Mo_A\ W_B\ V_C\ Nb_D\ Ta_E\ O_Y \quad (II),$$

mit

1/(A+B+C+D+E) 0,7 bis 1,3,

(B+C+D+E)/A 0,01 bis 1 und

Y eine Zahl, die durch die Wertigkeit und Häufigkeit der von Sauerstoff verschiedenen Elemente in II bestimmt wird,

eines neuen Strukturtyps (HT-Cu-Molybdat-Typ) enthalten, der durch das nachfolgende Röntgenbeugungsmuster, wiedergegeben in Gestalt von von der Wellenlänge der verwendeten Röntgenstrahlung unabhängigen Netzebenenabständen d [Å], definiert wird:

| d [Å] |
|---|
| 6,79 ± 0,3 |
| 3,56 ± 0,3 |
| 3,54 ± 0,3 |
| 3,40 ± 0,3 |
| 3,04 ± 0,3 |
| 2,96 ± 0,3 |
| 2,67 ± 0,2 |
| 2,66 ± 0,2 |
| 2,56 ± 0,2 |

(fortgesetzt)

| d [Å] |
|---|
| 2,36 ± 0,2 |
| 2,35 ± 0,2 |
| 2,27 ± 0,2 |
| 2,00 ± 0,2 |
| 1,87 ± 0,3 |
| 1,70 ± 0,2 |
| 1,64 ± 0,2 |
| 1,59 ± 0,2 |
| 1,57 ± 0,2 |
| 1,57 ± 0,2 |
| 1,55 ± 0,2 |
| 1,51 ± 0,2 |
| 1,44 ± 0,2. |

**2.** Multimetalloxidmassen nach Anspruch 1, mit $X^1$ = W, Nb und/oder Cr.

**3.** Multimetalloxidmassen nach Anspruch 1 oder 2, mit $X^2$ = Cu, Ni, Co und/oder Fe.

**4.** Multimetalloxidmassen nach Anspruch 1 bis 3, mit $X^3$ = Sb.

**5.** Multimetalloxidmassen nach Anspruch 1 bis 4, mit $X^4$ = Na und/oder K.

**6.** Multimetalloxidmassen nach Anspruch 1 bis 5, mit $X^5$ = Ca, Sr und/oder Ba.

**7.** Multimetalloxidmassen nach Anspruch 1 bis 6, mit $X^6$ = Si, Al und/oder Ti.

**8.** Multimetalloxidmassen nach Anspruch 1 bis 7, mit $X^7$ = Mo und/oder W.

**9.** Multimetalloxidmassen nach Anspruch 1 bis 8, mit a = 2 bis 6.

**10.** Multimetalloxidmassen nach Anspruch 1 bis 9, mit b = 0,5 bis 2,5.

**11.** Multimetalloxidmassen nach Anspruch 1 bis 10, mit c = 0 bis 4.

**12.** Multimetalloxidmassen nach Anspruch 1 bis 11, mit d = 0 bis 3.

**13.** Multimetalloxidmassen nach Anspruch 1 bis 12, mit e = 0 bis 0,3.

**14.** Multimetalloxidmassen nach Anspruch 1 bis 13, mit f = 0 bis 2.

**15.** Multimetalloxidmassen nach Anspruch 1 bis 14, mit g = 0 bis 20.

**16.** Multimetalloxidmassen nach Anspruch 1 bis 15, mit h = 6 bis 24.

**17.** Multimetalloxidmassen nach Anspruch 1 bis 16, mit h = 9 bis 17.

**18.** Multimetalloxidmassen nach Anspruch 1 bis 17, mit i = 0 bis 10.

**19.** Multimetalloxidmassen nach Anspruch 1 bis 18, mit p/q = 20:1 bis 1:1.

**20.** Multimetalloxidmassen nach Anspruch 1 bis 18, mit p/q = 15:1 bis 4:1.

**21.** Multimetalloxidmassen nach Anspruch 1 bis 20, deren Bereiche A eine Zusammensetzung gemäß der nachfol-

genden Formel IV

$$Mo_{12} V_{a'} X^1_{b'} X^2_{c'} X^5_{f'} X^6_{g'} O_{x'} \quad (IV),$$

mit

$X^1$ W und/oder Nb,
$X^2$ Cu und/oder Ni,
$X^5$ Ca und/oder Sr,
$X^6$ Si und/oder Al,
a' 2 bis 6,
b' 1 bis 2,
c' 1 bis 3,
f' 0 bis 0,75,
g' 0 bis 10 und
x' eine Zahl, die durch die Wertigkeit und Häufigkeit der von Sauerstoff verschiedenen Elementen in IV bestimmt wird,

aufweisen.

**22.** Multimetalloxidmassen nach Anspruch 1 bis 21, die den Anteil $[B]_q$ in Form dreidimensional ausgedehnter Bereiche der chemischen Zusammensetzung B enthalten, deren Größtdurchmesser $d_B$ > 0 bis 300 µm betragen.

**23.** Multimetalloxidmassen nach Anspruch 1 bis 21, die den Anteil $[B]_q$ in Form dreidimensional ausgedehnter Bereiche der chemischen Zusammensetzung B enthalten, deren Größtdurchmesser $d_B$ 0,1 bis 200 µm betragen.

**24.** Multimetalloxidmassen nach Anspruch 1 bis 21, die den Anteil $[B]_q$ in Form dreidimensional ausgedehnter Bereiche der chemischen Zusammensetzung B enthalten, deren Größtdurchmesser $d_B$ 0,5 bis 50 µm betragen.

**25.** Multimetalloxidmassen nach Anspruch 1 bis 21, die den Anteil $[B]_q$ in Form dreidimensional ausgedehnter Bereiche der chemischen Zusammensetzung B enthalten, deren Größtdurchmesser $d_B$ 1 bis 30 µm beträgt.

**26.** Multimetalloxidmassen nach Anspruch 1 bis 25, mit 1/(A+B+C+D+E) = 0,85 bis 1,15.

**27.** Multimetalloxidmassen nach Anspruch 1 bis 25, mit 1/(A+B+C+D+E) = 0,95 bis 1,05.

**28.** Multimetalloxidmassen nach Anspruch 1 bis 25, mit 1/(A+B+C+D+E) = 1.

**29.** Multimetalloxidmassen nach Anspruch 1 bis 28, mit (B+C+D+E)/A = 0,05 bis 0,3.

**30.** Multimetalloxidmassen nach Anspruch 1 bis 28, mit (B+C+D+E)/A = 0,075 bis 0,15.

**31.** Multimetalloxidmassen nach Anspruch 1 bis 28, mit (B+C+D+E)/A = 0,11.

**32.** Multimetalloxidmassen nach Anspruch 1 bis 31 mit C+D+E = 0.

**33.** Multimetalloxidmassen nach Anspruch 1 bis 25, wobei das Oxometallat II die Zusammensetzung $Cu_1Mo_{0,9}W_{0,1}O_{3,5-4}$ aufweist.

**34.** Multimetalloxidmassen nach Anspruch 1 bis 33, deren Bereiche B zusätzlich Kristallite enthalten, die den Strukturtyp eines oder mehrerer der nachfolgend aufgelisteten Kupfermolybdate aufweisen:

$Cu_3 (MoO_4)_2 (OH)_2$ (Lindgrenit, Karteikarte 36-405 der JCPDS-ICDD Kartei (1991)),

$Cu_4 MoO_6 O_{20}$ (A. Moini et al., Inorg. Chem. 25 (21) (1986) S. 3782 bis 3785),

| | |
|---|---|
| $Cu_4\ Mo_5\ O_{17}$ | (Karteikarte 39-181 der JCPDS-ICDD Kartei (1991)), |
| $Cu_6\ Mo_5\ O_{18}$ | (Karteikarte 40-865 der JCPDS-ICDD Kartei (1991)), |
| $Cu_6\ Mo_4\ O_{15}$ | (Karteikarte 35-17 der JCPDS-ICDD Kartei (1991)), |
| $Cu\ Mo\ O_4$ | (Karteikarte 22-242 der JCPDS-ICDD Kartei (1991)), |
| $Cu\ Mo\ O_4$ | (Russian Journal of Inorganic Chemistry 36 (7), 1991, S. 927-928, Table 1, $CuMoO_4$-III mit verzerrter Wolframit-Struktur ($CuWO_4$, Karteikarte 21-307 JCPDS-ICDD Kartei (1994)), |
| $Cu_{4-x}\ Mo_3\ O_{12}$ | mit x = O bis 0,25 (Karteikarte 24-56 und 26-547 der JCPDS-ICDD Kartei (1991)), |
| $Cu_3\ Mo_2\ O_9$ | (Karteikarte 24-55 und 34-637 der JCPDS-ICDD Kartei (1991)), |
| $Cu_2\ Mo\ O_5$ | (Karteikarte 22-607 der JCPDS-ICDD Kartei (1991)). |

**35.** Multimetalloxidmassen nach Anspruch 1 bis 33, deren Bereiche B zusätzlich Kristallite B** von Oxometallaten der allgemeinen Formel III

$$CuMo_{A'}\ W_{B'}\ V_{C'}\ Nb_{D'}Ta_{E'}\ O_{Y'}\cdot (H_2O)_{F'} \qquad \text{(III),}$$

mit

| | |
|---|---|
| 1/(A'+B'+C'+D'+E') | 0,7 bis 1,3, |
| F' | 0 bis 1, |
| B'+C'+D'+E' | 0 bis 1 und |
| Y' | eine Zahl, die durch die Wertigkeit und Häufigkeit der von Sauerstoff verschiedenen Elementen in III bestimmt wird, |

des Strukturtyps enthalten, der durch die Verbindung Cu-$MoO_4$-III in Russian Journal of Inorganic Chemistry 36 (7),1991 auf S. 921 in Table 1 definiert wird (verzerrter Wolframit-Typ).

**36.** Multimetalloxidmassen nach Anspruch 35, deren Kristallite von Oxometallaten III die Stöchiometrie VI

$$CuMo_{A'}\ W_{B'}V_{C'}O_{Y'} \qquad \text{(VI),}$$

mit

| | |
|---|---|
| 1/(A'+B'+C') | 0,7 bis 1,3, |
| A',B',C' | alle > 0, mit der Maßgabe, daß B'+C' ≤ 1 und |
| Y' | eine Zahl, die durch die Wertigkeit und Häufigkeit der von Sauerstoff verschiedenen Elemente in VI bestimmt wird, |

und/oder die Stöchiometrie VII

$$CuMo_{A'}\ W_{B'}O_{Y'} \qquad \text{(VII),}$$

mit

| | |
|---|---|
| 1/(A'+B') | 0,7 bis 1,3, |
| B'/A' | 0,01 bis 1 und |
| Y' | eine Zahl, die durch die Wertigkeit und Häufigkeit der von Sauerstoff verschiedenen Elemente in VII bestimmt wird, |

aufweisen.

**37.** Multimetalloxidmassen nach Anspruch 1 bis 36, deren Bereiche B ausschließlich Kristallite B* von Oxometallaten II und Kristallite B** von Oxometallaten III enthalten.

**38.** Multimetalloxidmassen nach Anspruch 1 bis 37, deren Anteil an Kristalliten B*, bezogen auf die Gesamtmasse des Anteils $[B]_{q,}$ > 0 bis 100, oder 1 bis 95, oder 5 bis 90, oder 10 bis 85, oder 15 bis 75, oder 25 bis 65, oder 35 bis 55, oder 40 bis 50 Gew.-% beträgt.

**39.** Oxometallate der allgemeinen Formel II

$$Cu\ Mo_A\ W_B\ V_C\ Nb_D\ Ta_E\ O_Y \quad (II),$$

mit

| | |
|---|---|
| 1/(A+B+C+D+E) | 0,7 bis 1,3, |
| (B+C+D+E)/A | 0,01 bis 1 und |
| Y | eine Zahl, die durch die Wertigkeit und Häufigkeit der von Sauerstoff verschiedenen Elemente in II bestimmt wird, |

deren Strukturtyp der durch nachfolgendes Röntgenbeugungsmuster, wiedergegeben in Gestalt von von der Wellenlänge der verwendeten Röntgenstrahlung unabhängigen Netzebenenabständen d [Å] definierte Strukturtyp (HT-Cu-Molybdat-Typ) ist:

| d [Å] |
|---|
| 6,79 ± 0,3 |
| 3,56 ± 0,3 |
| 3,54 ± 0,3 |
| 3,40 ± 0,3 |
| 3,04 ± 0,3 |
| 2,96 ± 0,3 |
| 2,67 ± 0,2 |
| 2,66 ± 0,2 |
| 2,56 ± 0,2 |
| 2,36 ± 0,2 |
| 2,35 ± 0,2 |
| 2,27 ± 0,2 |
| 2,00 ± 0,2 |
| 1,87 ± 0,2 |
| 1,70 ± 0,2 |
| 1,64 ± 0,2 |
| 1,59 ± 0,2 |
| 1,57 ± 0,2 |

(fortgesetzt)

| d [Å] |
|---|
| 1,57 ± 0,2 |
| 1,55 ± 0,2 |
| 1,51 ± 0,2 |
| 1,44 ± 0,2. |

**40.** Oxometallate nach Anspruch 39, mit 1/(A+B+C+D+E) = 0,85 bis 1,15.

**41.** Oxometallate nach Anspruch 39, mit 1/(A+B+C+D+E) = 0,95 bis 1,05.

**42.** Oxometallate nach Anspruch 39, mit 1/(A+B+C+D+E) = 1.

**43.** Oxometallate nach Anspruch 39 bis 42, mit (B+C+D+E)/A = 0,05 bis 2.

**44.** Oxometallate nach Anspruch 39 bis 42, mit (B+C+D+E)/A = 0,075 bis 0,15.

**45.** Oxometallate nach Anspruch 39 bis 42, mit (B+C+D+E)/A = 0,11.

**46.** Oxometallate nach Anspruch 39 bis 45, mit C+D+E = 0.

**47.** Oxometallate nach Anspruch 39 der Zusammensetzung $Cu_1Mo_{0,9}W_{0,1}O_{3,5\text{-}4}$.

**48.** Mischungen aus Oxometallaten gemäß Anspruch 39 bis 47 und Oxometallaten der allgemeinen Formel III

$$CuMo_{A'} W_{B'} V_{C'} Nb_{D'} Ta_{E'} O_{Y'} \cdot (H_2O)_{F'} \qquad \text{(III)},$$

mit

| | |
|---|---|
| 1/(A'+B'+C'+D'+E') | 0,7 bis 1,3, |
| F' | 0 bis 1 |
| B'+C'+D'+E' | 0 bis 1 und |
| Y' | eine Zahl, die durch die Wertigkeit und Häufigkeit der von Sauerstoff verschiedenen Elemente in III bestimmt wird, |

die den Wolframit-Strukturtyp aufweisen.

**49.** Verfahren zur Herstellung von Oxometallaten gemäß Anspruch 39 bis 48, dadurch gekennzeichnet, daß man Quellen der die Oxometallate konstituierenden Elemente miteinander innig mischt und das resultierende innige Gemisch an Luft oder unter Inertgas bei 700 bis 900°C calciniert und Oxometallate erhältlich nach diesem Verfahren.

**50.** Verfahren zur Herstellung von Cu, Mo und wenigstens eines der Elemente W, V, Nb und Ta enthaltenden Multimetalloxidmassen, dadurch gekennzeichnet, daß man als Ausgangsverbindung ein Oxometallat gemäß Anspruch 39 bis 48 mitverwendet.

**51.** Verwendung von Oxometallaten gemäß Anspruch 39 bis 48 zur Herstellung von Multimetalloxidmassen gemäß Anspruch 1 bis 38.

**52.** Verfahren zur Herstellung von Multimetalloxidmassen gemäß Anspruch 1 bis 38, dadurch gekennzeichnet, daß man ein Oxometallat

B $X^7_{12} Cu_h H_i O_y$ ,

das Kristallite B* enthält, in feinteiliger Form getrennt vorbildet (Ausgangsmasse 1) und anschließend die Ausgangsmasse 1 mit geeigneten Quellen der elementaren Konstituenten eines Multimetalloxids A

A $Mo_{12} V_a X^1_b X^2_c X^3_d X^4_e X^5_f X^6_g O_x$

im gewünschten Mengenverhältnis in innigen Kontakt bringt und ein daraus resultierendes Trockengemisch bei einer Temperatur von 250 bis 500°C calciniert.

**53.** Multimetalloxidmassen, erhältlich nach einem Verfahren gemäß Anspruch 52.

**54.** Verwendung von Multimetalloxidmassen gemäß Anspruch 1 bis 38 als Katalysatoren zur gasphasenkatalytisch oxidativen Herstellung von Acrylsäure aus Acrolein.

**55.** Verfahren der gasphasenkatalytisch oxidativen Herstellung von Acrylsäure aus Acrolein, dadurch gekennzeichnet, daß als Katalysator eine Multimetalloxidmasse gemäß Anspruch 1 bis 38 mitverwendet wird.

**56.** Cu, Mo und wenigstens eines der Elemente W, V, Nb und Ta enthaltende Multimetalloxidmassen mit einer mehrphasigen Struktur, enthaltend Kristallite B* von Oxometallaten II.

**57.** Verfahren der gasphasenkatalytischen Oxidation organischer Verbindungen, dadurch gekennzeichnet, daß als Katalysator eine Multimetalloxidmasse gemäß Anspruch 56 eingesetzt wird.

## Claims

**1.** A multimetal oxide material of the formula I

$$[A]_p [B]_q \qquad (I),$$

where

A is $Mo_{12} V_a X^1_b X^2_c X^3_d X^4_e X^5_f X^6_g O_x$ (active phase),

B is $X^7_{12} Cu_h H_i O_y$ (promoter phase),

$X^1$ is W, Nb, Ta, Cr or Ce,

$X^2$ is Cu, Ni, Co, Fe, Mn or Zn,

$X^3$ is Sb or Bi,

$X^4$ is Li, Na, K, Rb, Cs or H,

$X^5$ is Mg, Ca, Sr or Ba,

$X^6$ is Si, Al, Ti or Zr,

$X^7$ is Mo, W, V, Nb or Ta,

a is from 1 to 8,

b is from 0.2 to 5,

c is from 0 to 23,

d  is from 0 to 50,

e  is from 0 to 2,

f  is from 0 to 5,

g  is from 0 to 50,

h  is from 4 to 30,

i  is from 0 to 20,

x and y  are each numbers which are determined by the valency and frequency of the elements other than oxygen in I and

p and q  are non-zero numbers whose ratio p/q is from 160:1 to 1:1,

which contain the component $[A]_p$ in the form of three-dimensional regions A which are delimited with respect to their local environment owing to their chemical composition differing from their local environment and are of the chemical composition

A $Mo_{12} V_a X^1_b X^2_c X^3_d X^4_e X^5_f X^6_g O_x$

and the component $[B]_q$ in the form of three-dimensional regions B which are delimited from their local environment owing to their chemical composition differing from their local environment and are of the chemical composition

B $X^7_{12} Cu_h H_i O_y$

where the regions A and B are distributed relative to one another as in a mixture of finely divided A and finely divided B, with the proviso that the regions B contain crystallites B* of oxometallates of the formula II

$$Cu\, Mo_A\, W_B\, V_C\, Nb_D\, Ta_E\, O_Y \quad \text{(II)},$$

where

1/(A+B+C+D+E)  is from 0.7 to 1.3,

(B+C+D+E)/A  is from 0.01 to 1, and

Y  is a number which is determined by the valency and frequency of the elements other than oxygen in II,

of a novel structure type (HT Cu molybdate type) which is defined by the following X-ray diffraction pattern, reproduced in the form of interplanar spacings d[Å] independent of the wavelength of the X-rays used:

| d [Å] |
|---|
| 6.79 ± 0.3 |
| 3.56 ± 0.3 |
| 3.54 ± 0.3 |
| 3.40 ± 0.3 |
| 3.04 ± 0.3 |
| 2.96 ± 0.3 |
| 2.67 ± 0.2 |
| 2.66 ± 0.2 |
| 2.56 ± 0.2 |

(continued)

| d [Å] |
|---|
| 2.36 ± 0.2 |
| 2.35 ± 0.2 |
| 2.27 ± 0.2 |
| 2.00 ± 0.2 |
| 1.87 ± 0.3 |
| 1.70 ± 0.2 |
| 1.64 ± 0.2 |
| 1.59 ± 0.2 |
| 1.57 ± 0.2 |
| 1.57 ± 0.2 |
| 1.55 ± 0.2 |
| 1.51 ± 0.2 |
| 1.44 ± 0.2. |

**2.** A multimetal oxide material as claimed in claim 1, where $X^1$ is W, Nb or Cr.

**3.** A multimetal oxide material as claimed in claim 1 or 2, where $X^2$ is Cu, Ni, Co or Fe.

**4.** A multimetal oxide material as claimed in any of claims 1 to 3, where $X^3$ is Sb.

**5.** A multimetal oxide material as claimed in any of claims 1 to 4, where $X^4$ is Na or K.

**6.** A multimetal oxide material as claimed in any of claims 1 to 5, where $X^5$ is Ca, Sr or Ba.

**7.** A multimetal oxide material as claimed in any of claims 1 to 6, where $X^6$ is Si, Al or Ti.

**8.** A multimetal oxide material as claimed in any of claims 1 to 7, where $X^7$ is Mo or W.

**9.** A multimetal oxide material as claimed in any of claims 1 to 8, where a is from 2 to 6.

**10.** A multimetal oxide material as claimed in any of claims 1 to 9, where b is from 0.5 to 2.5.

**11.** A multimetal oxide material as claimed in any of claims 1 to 10, where c is from 0 to 4.

**12.** A multimetal oxide material as claimed in any of claims 1 to 11, where d is from 0 to 3.

**13.** A multimetal oxide material as claimed in any of claims 1 to 12, where e is from 0 to 0.3.

**14.** A multimetal oxide material as claimed in any of claims 1 to 13, where f is from 0 to 2.

**15.** A multimetal oxide material as claimed in any of claims 1 to 14, where g is from 0 to 20.

**16.** A multimetal oxide material as claimed in any of claims 1 to 15, where h is from 6 to 24.

**17.** A multimetal oxide material as claimed in any of claims 1 to 16, where h is from 9 to 17.

**18.** A multimetal oxide material as claimed in any of claims 1 to 17, where i is from 0 to 10.

**19.** A multimetal oxide material as claimed in any of claims 1 to 18, where p/q is from 20:1 to 1:1.

**20.** A multimetal oxide material as claimed in any of claims 1 to 18, where p/q is from 15:1 to 4:1.

**21.** A multimetal oxide material as claimed in any of claims 1 to 20, whose regions A have a composition according

to the following formula IV

$$Mo_{12}\, V_{a},\, X_b^1\, X_c^2\, X_f^5\, X_g^6\, O_x \qquad (IV),$$

where

$X^1$ is W or Nb,
$X^2$ is Cu or Ni,
$X^5$ is Ca or Sr,
$X^6$ is Si or Al,
a' is from 2 to 6,
b' is from 1 to 2,
c' is from 1 to 3,
f' is from 0 to 0.75,
g' is from 0 to 10 and
x' is a number which is determined by the valency and frequency of the elements other than oxygen in IV.

**22.** A multimetal oxide material as claimed in any of claims 1 to 21, which contains the component [B]q in the form of three-dimensional regions of chemical composition B whose maximum diameters $d_B$ are from > 0 to 300 µm.

**23.** A multimetal oxide material as claimed in any of claims 1 to 21, which contains the component [B]q in the form of three-dimensional regions of chemical composition B whose maximum diameters $d_B$ are from 0.1 to 200 µm.

**24.** A multimetal oxide material as claimed in any of claims 1 to 21, which contains the component [B]q in the form of three-dimensional regions of chemical composition B whose maximum diameters $d_B$ are from 0.5 to 50 µm.

**25.** A multimetal oxide material as claimed in any of claims 1 to 21, which contains the component [B]q in the form of three-dimensional regions of chemical composition B whose maximum diameters $d_B$ are from 1 to 30 µm.

**26.** A multimetal oxide material as claimed in any of claims 1 to 25, where 1/(A+B+C+D+E) is from 0.85 to 1.15.

**27.** A multimetal oxide material as claimed in any of claims 1 to 25, where 1/(A+B+C+D+E) is from 0.95 to 1.05.

**28.** A multimetal oxide material as claimed in any of claims 1 to 25, where 1/(A+B+C+D+E) is 1.

**29.** A multimetal oxide material as claimed in any of claims 1 to 28, where (B+C+D+E)/A is from 0.05 to 0.3.

**30.** A multimetal oxide material as claimed in any of claims 1 to 28, where (B+C+D+E)/A is from 0.075 to 0.15.

**31.** A multimetal oxide material as claimed in any of claims 1 to 28, where (B+C+D+E)/A is 0.11.

**32.** A multimetal oxide material as claimed in any of claims 1 to 31, where C+D+E is 0.

**33.** A multimetal oxide material as claimed in any of claims 1 to 25, wherein the oxometallate II has the composition $Cu_1Mo_{0.9}W_{0.1}O_{3.5\text{-}4}$.

**34.** A multimetal oxide material as claimed in any of claims 1 to 33, whose regions B additionally contain crystallites which have the structure type of one or more of the copper molybdates listed below:

$Cu_3\,(MoO_4)_2\,(OH)_2$ (lindgrenite, index card 36-405 of the JCPDS-ICDD index (1991)),

$Cu_4\,MoO_6\,O_{20}$ (A. Moini et al., Inorg. Chem. 25 (21) (1986) pages 3782 to 3785),

$Cu_4\,Mo_5\,O_{17}$ (index card 39-181 of the JCPDS-ICDD index (1991)),

$Cu_6\,Mo_5\,O_{18}$ (index card 40-865 of the JCPDS-ICDD index (1991)),

| | |
|---|---|
| $Cu_6\,Mo_4\,O_{15}$ | (index card 35-17 of the JCPDS-ICDD index (1991)), |
| $Cu\,Mo\,O_4$ | (index card 22-242 of the JCPDS-ICDD index (1991)), |
| $Cu\,Mo\,O_4$ | (Russian Journal of Inorganic Chemistry 36 (7), (1991), 927-928, Table 1, Cu Mo $O_4$-III with distorted wolframite structure ($CuWO_4$, index card 21-307 of the JCPDS-ICDD index (1994)), |
| $Cu_{4-x}\,Mo_3\,O_{12}$ | where x = 0 to 0.25 (index cards 24-56 and 26-547 of the JCPDS-ICDD index (1991)), |
| $Cu_3\,Mo_2\,O_9$ | (index cards 24-55 and 34-637 of the JCPDS-ICDD index (1991)), |
| $Cu_2\,Mo\,O_5$ | (index card 22-607 of the JCPDS-ICDD index (1991)). |

**35.** A multimetal oxide material as claimed in any of claims 1 to 33, whose regions B additionally contain crystallites B** of oxometallates of the formula III

$$CuMo_{A'}W_{B'}V_{C'}Nb_{D'}Ta_{E'}O_{y'}\cdot(H_2O)_{F'} \qquad \text{(III)},$$

where

| | |
|---|---|
| 1/(A'+B'+C'+D'+E') | is from 0.7 to 1.3, |
| F' | is from 0 to 1, |
| B'+C'+D'+E' | is from 0 to 1 and |
| Y' | is a number which is determined by the valency and frequency of the elements other than oxygen in III, |

of the structure type which is defined by the compound Cu-$MoO_4$-III in Russian Journal of Inorganic Chemistry 36 (7) (1991), 921, in Table 1 (distorted wolframite type).

**36.** A multimetal oxide material as claimed in claim 35, whose crystallites of oxometallates III have the stoichiometry VI

$$CuMo_{A'}W_{B'}V_{C'}O_{Y'} \qquad \text{(VI)},$$

where

| | |
|---|---|
| 1/(A'+B'+C') | is from 0.7 to 1.3, |
| A', B' and C' | are all > 0, with the proviso that B'+C' is ≤ 1, and |
| Y' | is a number which is determined by the valency and frequency of the elements other than oxygen in VI, |

or the stoichiometry VII

$$CuMo_{A'}W_{B'}O_{y'} \qquad \text{(VII)},$$

where

1/(A'+B') is from 0.7 to 1.3,

B'/A' is from 0.01 to 1 and

Y' is a number which is determined by the valency and frequency of the elements other than oxygen in VII.

**37.** A multimetal oxide material as claimed in any of claims 1 to 36, whose regions B contain exclusively crystallites B* of oxometallates II and crystallites B** of oxometallates III.

**38.** A multimetal oxide material as claimed in any of claims 1 to 37, in which the amount of crystallites B* is from > 0 to 100 or from 1 to 95 or from 5 to 90 or from 10 to 85 or from 15 to 75 or from 25 to 65 or from 35 to 55 or from 40 to 50 % by weight, based on the total weight of the component $[B]_q$.

**39.** An oxometallate of the formula II

$$Cu\ Mo_A\ W_B\ V_C\ Nb_D\ Ta_E\ O_Y \quad (II),$$

where

1/(A+B+C+D+E) is from 0.7 to 1.3,

(B+C+D+E)/A is from 0.01 to 1 and

Y is a number which is determined by the valency and frequency of the elements other than oxygen in II,

whose structure type is the (HT Cu molybdate type) defined by the following X-ray diffraction pattern, reproduced in the form of interplanar spacings d[Å] independent of the wavelength of the X-rays used:

| d [Å] |
|---|
| 6.79 ± 0.3 |
| 3.56 ± 0.3 |
| 3.54 ± 0.3 |
| 3.40 ± 0.3 |
| 3.04 ± 0.3 |
| 2.96 ± 0.3 |
| 2.67 ± 0.2 |
| 2.66 ± 0.2 |
| 2.56 ± 0.2 |
| 2.36 ± 0.2 |
| 2.35 ± 0.2 |
| 2.27 ± 0.2 |
| 2.00 ± 0.2 |
| 1.87 ± 0.2 |
| 1.70 ± 0.2 |
| 1.64 ± 0.2 |
| 1.59 ± 0.2 |
| 1.57 ± 0.2 |
| 1.57 ± 0.2 |
| 1.55 ± 0.2 |
| 1.51 ± 0.2 |
| 1.44 ± 0.2. |

**40.** An oxometallate as claimed in claim 39, where 1/(A+B+C+D+E) is from 0.85 to 1.15.

**41.** An oxometallate as claimed in claim 39, where 1/(A+B+C+D+E) is from 0.95 to 1.05.

**42.** An oxometallate as claimed in claim 39, where 1/(A+B+C+D+E) is 1.

**43.** An oxometallate as claimed in any of claims 39 to 42, where (B+C+D+E)/A is from 0.05 to 2.

**44.** An oxometallate as claimed in any of claims 39 to 42, where (B+C+D+E)/A is from 0.075 to 0.15.

**45.** An oxometallate as claimed in any of claims 39 to 42, where (B+C+D+E)/A is 0.11.

**46.** An oxometallate as claimed in any of claims 39 to 45, where C+D+E is 0.

**47.** An oxometallate as claimed in claim 39, having the composition $Cu_1Mo_{0.9}W_{0.1}O_{3.5-4}$.

**48.** A mixture of oxometallates as claimed in any of claims 39 to 47 and oxometallates of the formula III

$$CuMo_{A}, W_{B}, V_{C}, Nb_{D}, Ta_{E}, O_{Y}, \cdot (H_2O)_{F}, \qquad (III),$$

where

| | |
|---|---|
| 1/(A'+B'+C'+D'+E') | is from 0.7 to 1.3, |
| F' | is from 0 to 1 |
| B'+C'+D'+E' | is from 0 to 1 and |
| Y' | is a number which is determined by the valency and frequency of the elements other than oxygen in III, |

which have the wolframite structure type.

**49.** A process for the preparation of oxometallates as claimed in any of claims 39 to 48, wherein sources of the elements constituting the oxometallates are thoroughly mixed with one another and the resulting intimate mixture is calcined in air or under inert gas at from 700 to 900°C, and oxometallates obtainable by this process.

**50.** A process for the preparation of multimetal oxide materials containing Cu, Mo and at least one of the elements W, V, Nb and Ta, wherein an oxometallate as claimed in any of claims 39 to 48 is present as a starting compound.

**51.** Use of an oxometallate as claimed in any of claims 39 to 48 for the preparation of multimetal oxide materials as claimed in any of claims 1 to 38.

**52.** A process for the preparation of multimetal oxide materials as claimed in any of claims 1 to 38, wherein an oxometallate

B $X^7_{12}\, Cu_h\, H_i\, O_y$ ,

which contains crystallites B* is pre-formed in finely divided form (starting material 1) and the starting material 1 is then brought into intimate contact with suitable sources of the elemental constituents of a multimetal oxide A

A $Mo_{12}\, V_a\, X^1_b\, X^2_c\, X^3_d\, X^4_e\, X^5_f\, X^6_g\, O_x$

in the desired ratio, and a resulting dry mixture is calcined at from 250 to 500°C.

**53.** A multimetal oxide material obtainable by a process as claimed in claim 52.

**54.** Use of a multimetal oxide material as claimed in any of claims 1 to 38 as a catalyst for the preparation of acrylic

acid from acrolein by gas-phase catalytic oxidation.

**55.** A process for the preparation of acrylic acid from acrolein by gas-phase catalytic oxidation, wherein a multimetal oxide material as claimed in any of claims 1 to 38 is concomitantly used as the catalyst.

**56.** A multimetal oxide material containing Cu, Mo and at least one of the elements W, V, Nb and Ta, having a multiphase structure and containing crystallites B* of oxometallates II.

**57.** A process for the gas-phase catalytic oxidation of organic compounds, wherein a multimetal oxide material as claimed in claim 56 is used as the catalyst.

**Revendications**

**1.** Masses d'oxydes multimétalliques de formule générale I

$$[A]_p\ [B]_q \qquad (I),$$

dans laquelle les variables ont le signification suivante :

| | |
|---|---|
| A | $Mo_{12}\ V_a\ X^1_b\ X^2_c\ X^3_d\ X^4_e\ X^5_f\ X^6_g\ O_x$ (phase active) |
| B | $X^7_{12}\ Cu_h\ H_i\ O_y$ (phase promoteur) |
| $X^1$ | représente W, Nb, Ta, Cr et/ou Ce, |
| $X^2$ | représente Cu, Ni, Co, Fe, Mn et/ou Zn, |
| $X^3$ | représente Sb et/ou Bi, |
| $X^4$ | représente Li, Na, K, Rb, Cs et/ou H, |
| $X^5$ | représente Mg, Ca, Sr et/ou Ba, |
| $X^6$ | représente Si, Al, Ti et/ou Zr, |
| $X^7$ | représente Mo, W, V, Nb et/ou Ta, |
| a | vaut 1-8, |
| b | vaut 0,2-5, |
| c | vaut 0-23, |
| d | vaut 0-50, |
| e | vaut 0-2, |
| f | vaut 0-5, |
| g | vaut 0-50, |
| h | vaut 4-30, |
| i | vaut 0-20, |
| x,y | représentent des nombres définis par la valence et la fréquence des éléments de I différents de l'oxygène et |
| p,q | représentent des nombres non nuls dont le rapport p/q vaut de 160:1 à 1:1, |

qui contiennent la partie $[A]_p$ sous forme de domaines A, ayant une extension tridimensionnelle, séparés de leur environnement local du fait de leur composition chimique, qui est différente de celle de leur environnement local, et ayant la composition chimique

A $Mo_{12}\ V_a\ X^1_b\ X^2_c\ X^3_d\ X^4_e\ X^5_f\ X^6_g\ O_x$

et la partie $[B]_q$ sous forme de domaines B, ayant une extension tridimensionnelle, séparés de leur environnement local du fait de leur composition chimique, qui est différente de celle de leur environnement local, et ayant la composition chimique :

B $X^7_{12}\ Cu_h\ H_i\ O_y$

où les domaines A, B sont répartis l'un par rapport à l'autre comme dans un mélange constitué de A finement divisé et de B finement divisé, étant spécifié que les domaines B contiennent des cristallites B* d'oxométallates de formule générale II

$$Cu\ Mo_A\ W_B\ V_C\ Nb_D\ Ta_E\ O_Y \quad (II),$$

dans laquelle

| | |
|---|---|
| 1/(A+B+C+D+E) vaut | 0,7-1,3, |
| (B+C+D+E)/A vaut | 0,01-1, et |
| Y | est un nombre qui est défini par la valence et la fréquence des éléments de II différents de l'oxygène, |

ayant un nouveau type de structure (type HT-molybdate de Cu), défini par le diagramme de diffraction X suivant, donné sous forme des distances entre plans du réseau d[Å], indépendantes des longueurs d'onde des rayons X utilisés :

| d [Å] |
|---|
| 6,79 ± 0,3 |
| 3,56 ± 0,3 |
| 3,54 ± 0,3 |
| 3,40 ± 0,3 |
| 3,04 ± 0,3 |
| 2,96 ± 0,3 |
| 2,67 ± 0,2 |
| 2,66 ± 0,2 |
| 2,56 ± 0,2 |
| 2,36 ± 0,2 |
| 2,35 ± 0,2 |
| 2,27 ± 0,2 |
| 2,00 ± 0,2 |
| 1,87 ± 0,3 |
| 1,70 ± 0,2 |
| 1,64 ± 0,2 |
| 1,59 ± 0,2 |
| 1,57 ± 0,2 |
| 1,57 ± 0,2 |
| 1,55 ± 0,2 |
| 1,51 ± 0,2 |
| 1,44 ± 0,2. |

**2.** Masses d'oxydes multimétalliques selon la revendication 1, dans lesquelles $X^1$ est W, Nb et/ou Cr.

**3.** Masses d'oxydes multimétalliques selon la revendication 1 ou 2, dans lesquelles $X^2$ est Cu, Ni, Co et/ou Fe.

**4.** Masses d'oxydes multimétalliques selon les revendications 1 à 3, dans lesquelles $X^3$ est Sb.

**5.** Masses d'oxydes multimétalliques selon les revendications 1 à 4, dans lesquelles $X^4$ est Na et/ou K.

**6.** Masses d'oxydes multimétalliques selon les revendications 1 à 5, dans lesquelles $X^5$ est Ca, Sr et/ou Ba.

**7.** Masses d'oxydes multimétalliques selon les revendications 1 à 6, dans lesquelles $X^6$ est Si, Al et/ou Ti.

**8.** Masses d'oxydes multimétalliques selon les revendications 1 à 7, dans lesquelles $X^7$ est Mo et/ou W.

**9.** Masses d'oxydes multimétalliques selon les revendications 1 à 8, dans lesquelles a vaut de 2 à 6.

**10.** Masses d'oxydes multimétalliques selon les revendications 1 à 9, dans lesquelles b vaut de 0,5 à 2,5.

**11.** Masses d'oxydes multimétalliques selon les revendications 1 à 10, dans lesquelles c vaut de 0 à 4.

**12.** Masses d'oxydes multimétalliques selon les revendications 1 à 11, dans lesquelles d vaut de 0 à 3.

**13.** Masses d'oxydes multimétalliques selon les revendications 1 à 12, dans lesquelles e vaut de 0 à 0,3.

**14.** Masses d'oxydes multimétalliques selon les revendications 1 à 13, dans lesquelles f vaut de 0 à 2.

**15.** Masses d'oxydes multimétalliques selon les revendications 1 à 14, dans lesquelles g vaut de 0 à 20.

**16.** Masses d'oxydes multimétalliques selon les revendications 1 à 15, dans lesquelles h vaut de 6 à 24.

**17.** Masses d'oxydes multimétalliques selon les revendications 1 à 16, dans lesquelles h vaut de 9 à 17.

**18.** Masses d'oxydes multimétalliques selon les revendications 1 à 17, dans lesquelles i vaut de 0 à 10.

**19.** Masses d'oxydes multimétalliques selon les revendications 1 à 18, dans lesquelles p/q vaut de 20:1 à 1:1.

**20.** Masses d'oxydes multimétalliques selon les revendications 1 à 18, dans lesquelles p/q vaut de 15:1 à 4:1.

**21.** Masses d'oxydes multimétalliques selon les revendications 1 à 20, dont les domaines A ont une composition selon la formule IV ci-après :

$$Mo_{12} V_a X^1_b X^2_c X^5_f X^6_g O_x, \quad (IV),$$

dans laquelle

$X^1$ est W et/ou Nb,
$X^2$ est Cu et/ou Ni,
$X^5$ est Ca et/ou Sr,
$X^6$ est Si et/ou Al,
a' vaut de 2 à 6,
b' vaut de 1 à 2,
c' vaut de 1 à 3,
f vaut de 0 à 0,75,
g' vaut de 0 à 10, et
x' est un nombre qui est défini par la valence et la fréquence des éléments de IV différents de l'oxygène.

**22.** Masses d'oxydes multimétalliques selon les revendications 1 à 21, qui contiennent la partie $[B]_q$ sous forme de domaines ayant une extension tridimensionnelle et ayant la composition chimique B, dont le diamètre maximal dB est compris entre > 0 et 300 µm.

**23.** Masses d'oxydes multimétalliques selon les revendications 1 à 21, qui contiennent la partie $[B]_q$ sous forme de domaines ayant une extension tridimensionnelle et ayant la composition chimique B, dont le diamètre maximal dB est compris entre 0,1 et 200 µm.

**24.** Masses d'oxydes multimétalliques selon les revendications 1 à 21, qui contiennent la partie $[B]_q$ sous forme de domaines ayant une extension tridimensionnelle et ayant la composition chimique B, dont le diamètre maximal dB est compris entre 0,5 et 50 µm.

**25.** Masses d'oxydes multimétalliques selon les revendications 1 à 21, qui contiennent la partie $[B]_q$ sous forme de domaines ayant une extension tridimensionnelle et ayant la composition chimique B, dont le diamètre maximal dB est compris entre 1 et 30 µm.

**26.** Masses d'oxydes multimétalliques selon les revendications 1 à 25, dans lesquelles 1/(A+B+C+D+E) vaut de 0,85 à 1,15.

**27.** Masses d'oxydes multimétalliques selon les revendications 1 à 25, dans lesquelles 1/(A+B+C+D+E) vaut de 0,95 à 1,05.

**28.** Masses d'oxydes multimétalliques selon les revendications 1 à 25, dans lesquelles 1/(A+B+C+D+E) vaut 1.

**29.** Masses d'oxydes multimétalliques selon les revendications 1 à 28, dans lesquelles (B+C+D+E)/A vaut de 0,05 à 0,3.

**30.** Masses d'oxydes multimétalliques selon les revendications 1 à 28, dans lesquelles (B+C+D+E)/A vaut de 0,075 à 0,15.

**31.** Masses d'oxydes multimétalliques selon les revendications 1 à 28, dans lesquelles (B+C+D+E)/A vaut 0,11.

**32.** Masses d'oxydes multimétalliques selon les revendications 1 à 31, dans lesquelles C+D+E vaut 0.

**33.** Masses d'oxydes multimétalliques selon les revendications 1 à 25, dans lesquelles l'oxométallate II présente la composition $Cu_1Mo_{0,9}W_{0,1}O_{3,5-4}$.

**34.** Masses d'oxydes multimétalliques selon les revendications 1 à 33, dont les domaines B contiennent en outre des cristallites qui présentent le type de structure d'un ou plusieurs des molybdates de cuivre ci-après:

| | |
|---|---|
| $Cu_3(MoO_4)_2(OH)_2$ | (lindgrenite, fiche 36-405 du fichier JCPDS-ICDD (1991)), |
| $Cu_4Mo_6O_{20}$ | (A. Moini et al., Inorg. Chem. 25 (21) (1986) p. 3782 à 3785), |
| $Cu_4Mo_5O_{17}$ | (fiche 39-181 du fichier JCPDS-ICDD (1991)), |
| $Cu_6Mo_5O_{18}$ | (fiche 40-865 du fichier JCPDS-ICDD (1991)), |
| $Cu_6Mo_4O_{15}$ | (fiche 35-17 du fichier JCPDS-ICDD (1991)), |
| $CuMoO_4$ | (fiche 22-242 du fichier JCPDS-ICDD (1991)), |
| $CuMoO_4$ | (Russian Journal of Inorganic Chemistry 36 (7), 1991, p. 927-928, Tableau 1, $CuMoO_4$-III avec une structure de wolframite déformée($CuWO_4$ fiche 21-307 du fichier JCPDS-ICDD (1994))), |
| $Cu_{4-x}Mo_3O_{12}$, | où x vaut de 0 à 0,25 (fiches 24-56 et 26-547 du fichier JCPDS-ICDD (1991)), |
| $Cu_3Mo_2O_9$ | (fiches 24-55 et 34-637 du fichier JCPDS-ICDD (1991)), |
| $Cu_2MoO_5$ | (fiche 22-607 du fichier JCPDS-ICDD (1991)). |

**35.** Masses d'oxydes multimétalliques selon les revendications 1 à 33, dont les zones B contiennent en outre des cristallites B** d'oxométallates de formule générale III

$$CuMo_{A'}, W_{B'}, V_{C'}, Nb_{D'}, Ta_{E'}, O_{Y'} \cdot (H_2O)_{F'}, \quad \text{(III)},$$

dans laquelle

| | |
|---|---|
| 1/(A'+B'+C'+D'+E') | vaut de 0,7 à 1,3, |
| F' | vaut de 0 à 1, |
| B'+C'+D'+E' | vaut de 0 à 1, et |
| Y' | est un nombre qui est défini par la valence et la fréquence des éléments de III différents de l'oxygène, |

ayant une structure du type défini par le composé $CuMoO_4$-III dans le "Russian Journal of Inorganic Chemistry" 36 (7), 1991, p. 921 dans le Tableau 1 (type wolframite déformée).

**36.** Masses d'oxydes multimétalliques selon la revendication 35, dont les cristallites d'oxométallates III présentent la stoechyométrie VI

$$CuMo_{A'}, W_{B'}, V_{C'}, O_{Y'}, \quad \text{(VI)},$$

dans laquelle

| | |
|---|---|
| 1/(A'+B'+C') | vaut de 0,7 à 1,3, |
| A', B' et C' | ont tous une valeur > 0, à la condition que B'+C'≤1, et |
| Y' | est un nombre qui est défini par la valence et la fréquence des éléments de VI différents de l'oxygène, |

et/ou la stoechyométrie VII

$$CuMo_{A'}W_{B'}O_{Y'} \qquad (VII),$$

dans laquelle

| | |
|---|---|
| 1/(A'+B') | vaut de 0,7 à 1,3, |
| B'/A' | vaut de 0,01 à 1 et |
| Y' | est un nombre qui est défini par la valence et la fréquence des éléments de VII différents de l'oxygène. |

**37.** Masses d'oxydes multimétalliques selon les revendications 1 à 36, dans lesquelles les domaines B contiennent exclusivement des cristallites B* d'oxométallates II et des cristallites B** d'oxométallates III.

**38.** Masses d'oxydes multimétalliques selon les revendications 1 à 37, dans lesquelles la proportion des cristallites B*, rapportée à la masse totale de la partie $[B]_q$, est > 0 à 100, ou de 1 à 95, ou de 5 à 90, ou de 10 à 85, ou de 15 à 75, ou de 25 à 65, ou de 35 à 55, ou de 40 à 50% en poids.

**39.** Oxométallates de formule générale II

$$Cu\,Mo_A\,W_B\,V_C\,Nb_D\,Ta_E\,O_Y \qquad (II),$$

dans laquelle

| | |
|---|---|
| 1/(A+B+C+D+E) | vaut de 0,7 à 1,3, |
| (B+C+D+E)/A | vaut de 0,01 à 1, et |
| Y | est un nombre qui est défini par la valence et la fréquence des éléments de II différents de l'oxygène, |

dont le type de structure est la structure (type HT-molybdate de Cu), définie par le diagramme de diffraction X suivant, donné sous forme des distances entre plans du réseau d[Å], indépendantes des longueurs d'onde des rayons X utilisés :

| d [Å] |
|---|
| 6,79 ± 0,3 |
| 3,56 ± 0,3 |
| 3,54 ± 0,3 |
| 3,40 ± 0,3 |
| 3,04 ± 0,3 |
| 2,96 ± 0,3 |
| 2,67 ± 0,2 |
| 2,66 ± 0,2 |
| 2,56 ± 0,2 |
| 2,36 ± 0,2 |
| 2,35 ± 0,2 |
| 2,27 ± 0,2 |
| 2,00 ± 0,2 |
| 1,87 ± 0,2 |

(suite)

| d [Å] |
|---|
| 1,70 ± 0,2 |
| 1,64 ± 0,2 |
| 1,59 ± 0,2 |
| 1,57 ± 0,2 |
| 1,57 ± 0,2 |
| 1,55 ± 0,2 |
| 1,51 ± 0,2 |
| 1,44 ± 0,2. |

**40.** Oxométallates selon la revendication 39, dans lesquels 1/(A+B+C+D+E) vaut de 0,85 à 1,15.

**41.** Oxométallates selon la revendication 39, dans lesquels 1/(A+B+C+D+E) vaut de 0,95 à 1,05.

**42.** Oxométallates selon la revendication 39, dans lesquels 1/(A+B+C+D+E) vaut 1.

**43.** Oxométallates selon les revendications 39 à 42, dans lesquelles (B+C+D+E)/A vaut de 0,05 à 2.

**44.** Oxométallates selon les revendications 39 à 42, dans lesquelles (B+C+D+E)/A vaut de 0,075 à 0,15.

**45.** Oxométallates selon les revendications 39 à 42, dans lesquelles (B+C+D+E)/A vaut 0,11.

**46.** Oxométallates selon les revendications 39 à 45, dans lesquelles C+D+E vaut 0.

**47.** Oxométallate selon la revendication 39 ayant la composition $Cu_1Mo_{0,9}W_{0,1}O_{3,5\text{-}4}$.

**48.** Mélanges d'oxométallates selon les revendications 39 à 47 et d'oxométallates de formule générale III

$$CuMo_{A'}W_{B'}V_{C'}Nb_{D'}Ta_{E'}O_{Y'} \cdot (H_2O)_{F'} \qquad \text{(III)},$$

dans laquelle

| | |
|---|---|
| 1/(A'+B'+C'+D'+E') | vaut de 0,7 à 1,3, |
| F' | vaut de 0 à 1, |
| B'+C'+D'+E' | vaut de 0 à 1, et |
| Y' | est un nombre qui est défini par la valence et la fréquence des éléments de III différents de l'oxygène, |

ayant une structure de type wolframite.

**49.** Procédé de préparation d'oxométallates selon les revendications 39 à 48, caractérisé en ce que l'on mélange intimement l'une avec l'autre des sources des éléments constituant les oxométallates, puis on calcine à l'air ou sous gaz inerte, le mélange intime obtenu à des températures de 700 à 900°C, et oxométallates pouvant être obtenus par ce procédé.

**50.** Procédé de préparation de masses d'oxydes multimétalliques contenant Cu, Mo et au moins un des éléments W, V, Nb et Ta, caractérisé en ce que l'on utilise comme composé de départ un oxométallate selon les recendications 39 à 48.

**51.** Utilisation d'oxométallates selon les revendications 39 à 48 pour préparer des masses d'oxydes multimétalliques selon les revendications 1 à 38.

**52.** Procédé de préparation de masses d'oxydes multimétaliques selon les revendications 1 à 38, caractérisé en ce que l'on prépare au préalable, de façon séparée et sous forme finement divisée, un oxométallate

B $X^7_{12}$ $Cu_h$ $H_i$ $O_y$ ,

contenant des cristallites B* (masse de départ 1) puis l'on met en contact intime la masse de départ 1 avec les sources adéquates des constituants élémentaires d'un oxyde multimétallique A

A $Mo_{12}$ $V_a$ $X^1_b$ $X^2_c$ $X^3_d$ $X^4_e$ $X^5_f$ $X^6_g$ $O_x$

dans le rapport en poids souhaité, et l'on calcine le mélange sec obtenu à une température de 250 à 500°C.

**53.** Masses d'oxydes multimétalliques pouvant être obtenues par un procédé selon la revendication 52.

**54.** Utilisation de masses d'oxydes multimétalliques selon les revendications 1 à 38 en tant que catalyseurs pour préparer de l'acide acrylique à partir d'acroléine par oxydation catalysée en phase gazeuse.

**55.** Procédé de préparation par oxydation catalysée en phase gazeuse d'acide acrylique à partir d'acroléine, caractérisé en ce qu'on utilise en tant que catalyseur une masse d'oxydes multimétalliques selon les revendications 1 à 38.

**56.** Masses d'oxydes multimétalliques contenant Cu, Mo et au moins un des éléments W, V, Nb et Ta ayant une structure polyphasique, contenant des cristallites B* d'oxométallates II.

**57.** Procédé d'oxydation catalytique en phase gazeuse de composés organiques, caractérisé en ce que l'on utilise comme catalyseur, une masse d'oxydes multimétalliques selon la revendication 56.